# EUROPÄISCHE PATENTSCHRIFT

(11) **EP 2 625 159 B1**
(45) Veröffentlichungstag und Bekanntmachung des Hinweises auf die Patenterteilung: **02.10.2024**
(21) Anmeldenummer: 11771064.0
(22) Anmeldetag: 04.10.2011
(51) Int. Cl.: C07C 51/50, C07C 57/04

(54) **VERFAHREN ZUR HEMMUNG DER UNERWÜNSCHTEN RADIKALISCHEN POLYMERISATION VON IN EINER FLÜSSIGEN PHASE P BEFINDLICHER ACRYLSÄURE**
PROCESS FOR INHIBITING UNWANTED FREE-RADICAL POLYMERIZATION OF ACRYLIC ACID PRESENT IN A LIQUID PHASE P
PROCÉDÉ POUR INHIBER LA POLYMÉRISATION RADICALAIRE INDÉSIRABLE D'ACIDE ACRYLIQUE SE TROUVANT DANS UNE PHASE LIQUIDE P

(30) Priorität: 08.10.2010 US 391102 P; 08.10.2010 DE 102010042216
(43) Veröffentlichungstag der Anmeldung: 14.08.2013
(73) Patentinhaber: BASF SE, 67056 Ludwigshafen am Rhein (DE)
(72) Erfinder: BLUM, Till, Singapore 238713 (SG); ZUROWSKI, Peter, 76829 Landau (DE); KORN, Tobias, Johannes, 67061 Ludwigshafen (DE); HAREMZA, Sylke, 69151 Neckargemünd (DE); FRIESE, Thorsten, 68199 Mannheim (DE); MARTIN, Friedrich-Georg, 69124 Heidelberg (DE); JÄGER, Ulrich, 67354 Römerberg (DE); RISSEL, Steffen, 67281 Kirchheim (DE); SCHLIEPHAKE, Volker, 67105 Schifferstadt (DE)
(74) Vertreter: BASF IP Association
(86) Internationale Anmeldenummer: PCT/EP2011/067305
(87) Internationale Veröffentlichungsnummer: WO 2012/045738

(56) Entgegenhaltungen:
- EP-A1- 1 396 484
- DE-A1- 102008 040 799
- US-A- 4 021 310
- US-A1- 2004 225 151

## Beschreibung

Vorliegende Erfindung betrifft ein Verfahren zur Hemmung der unerwünschten radikalischen Polymerisation von in einer flüssigen Phase P befindlicher Acrylsäure, deren Acrylsäuregehalt wenigstens 10 Gew.-% beträgt und die, bezogen auf das Gewicht der in ihr enthaltenen Acrylsäure, zusätzlich wenigstens 100 Gew.-ppm Propionsäure und wenigstens 100 Gew.-ppm Glyoxal enthält.

Acrylsäure ist ein bedeutendes Monomeres, das als solches, in Form seiner Salze und/oder in Form seiner Ester (z.B. Alkylester) zur Erzeugung von Polymerisaten Verwendung findet, die z.B. als Klebstoffe oder als Wasser superabsorbierende Materialien eingesetzt werden (vgl. z.B. WO 02/055469 und WO 03/078378).

Die Herstellung von Acrylsäure kann z.B. durch heterogen katalysierte partielle Oxidation einer C₃-Vorläuferverbindung (z.B. Propylen, Propan, Acrolein, Propionaldehyd, Propionsäure, Propanol und/oder Glyzerin) in der Gasphase erfolgen (vgl. z.B. WO 2010/012586, US-A 5,198,578, EP-A 1 710 227, EP-A 1 015 410, EP-A 1 484303, EP-A 1 484 308, EP-A 1 484 309, US-A 2004/0242826, WO 2006/136336, DE-A 10 028 582 und WO 2007/074044).

Grundsätzlich wird im Rahmen einer solchen heterogen katalysierten partiellen Gasphasenoxidation keine reine Acrylsäure, sondern lediglich ein Acrylsäure enthaltendes Produktgasgemisch erhalten, das neben Acrylsäure auch von Acrylsäure verschiedene Bestandteile enthält, von denen die Acrylsäure abgetrennt werden muss.

Sowohl die Art als auch der Mengenanteil der von Acrylsäure verschiedenen Bestandteile im Produktgasgemisch kann unter anderem durch die Wahl der C₃-Vorläuferverbidngung, durch den verwendeten Katalysator, durch die Reaktionsbedingungen, bei denen die heterogen katalysierte partielle Gasphasenoxidation durchgeführt wird, durch die Art und Menge der in der als Rohstoff eingesetzten C₃-Vorläuferberbindung enthaltenen, von der C₃-Vorläuferverbindung verschiedenen, Verunreinigungsbestandteile sowie durch die Auswahl der die Reaktanten im Reaktionsgasgemisch in der Regel verdünnenden Verdünnungsgase beeinflusst werden (vgl. z.B. DE-A 10 131 297, DE-A 10 2005 0529 17, WO 2007/074044 und DE-A 10 028 582).

Zur Abtrennung der Acrylsäure aus dem Produktgasgemisch der heterogen katalysierten partiellen Gasphasenoxidation einer C₃-Vorläuferverbindung wird normalerweise eine Kombination unterschiedlicher Trennverfahren angewendet, um auf möglichst wirtschaftliche Weise eine dem nachfolgenden Verwendungszweck der Acrylsäure angemessene Reinheit derselben zu erzielen. Die im Einzelnen angewandte Kombination ist dabei unter anderem von der Art und Menge der im Produktgasgemisch enthaltenen, von Acrylsäure verschiedenen Bestandteile abhängig.

Ein im Wesentlichen allen möglichen Kombinationen von Trennverfahren zur Abtrennung von Acrylsäure aus dem Produktgasgemisch einer heterogen katalysierten partiellen Gasphasenoxidation einer C₃-Vorläuferverbindung gemeinsames Merkmal ist, dass, gegebenenfalls nach direktem und/oder indirektem Abkühlen des vorgenannten Produktgasgemischs, im Produktgasgemisch enthaltene Acrylsäure in einem Grundabtrennschritt in die kondensierte (insbesondere flüssig) Phase überführt wird.

Dies kann z.B. durch Absorption in ein geeignetes Lösungsmittel (z.B. Wasser, hochsiedende organische Lösungsmittel, wässrige Lösungen) und/oder durch partielle oder im Wesentlichen vollständige Kondensation (z.B. fraktionierende Kondensation) erfolgen (vgl. dazu z.B. die Schriften EP-A 1 388 533, EP-A 1 388 532, DE-A 10 235 847, EP-A 792 867, WO 98/01415, US 7,332,624 B2, US 6,888,025 B2, US 7,109,372 B2, EP-A 1 015 411, EP-A 1 015 410, WO 99/50219, WO 00/53560, WO 02/09839, DE-A 10 235 847, WO 03/041832, DE-A 10 223 058, DE-A 10 243 625, DE-A 10 336 386, EP-A 854129, US 7,319,167 B2, US-A 4,317,926, DE-A 1 983 752 0, DE-A 1 960 687 7, DE-A 1 905 013 25, DE-A 10 247 240, DE-A 1 974 025 3, EP-A 695 736, EP-A 982 287, EP-A 1 041 062, EP-A 117 146, DE-A 4 308 087, DE-A 4 335 172, DE-A 4 436 243, DE-A 19 924 532, DE-A 10 332 758 sowie DE-A 19 924 533). Eine Acrylsäureabtrennung kann auch wie in der EP-A 982 287, der EP-A 982 289, der DE-A 10 336 386, der DE-A 10 115 277, der DE-A 19 606 877, der DE-A 19 740 252, der DE-A 19 627 847, der EP-A 920 408, der EP-A 10 681 74, der EP-A 10 662 39, der EP-A 10 662 40, der WO 00/53560, der WO 00/53561, der DE-A 10 053 086 und der EP-A 982 288 vorgenommen werden. Günstige Abtrennweisen sind auch die in den Schriften WO 2004/063138, WO 2008/090190, WO 2004/035514, DE-A 10 243 625 und DE-A 10 235 847 beschriebenen Verfahren.

Mit der Acrylsäure werden normalerweise auch von Acrylsäure verschiedene im Produktgasgemisch der heterogen katalysierten partiellen Gasphasenoxidation enthaltene Bestandteile in die kondensierte Phase überführt.

Aus den Schriften WO 2007/074044, WO 2007/074045 und DE-A 10 2007 029 053 ist bekannt, dass dann, wenn das Reaktionsgasgemisch Cyclopropan als Verunreinigung enthält, im Rahmen der heterogen katalysierten partiellen Gasphasenoxidation der C₃-Vorläuferverbindung (z.B. Propylen) zu Acrylsäure im Produktgasgemisch in der Regel erhöhte Mengen an Propionsäure als Nebenprodukt auftreten und diese Propionsäure bei der vorstehend beschriebenen Grundabtrennung der Acrylsäure aus dem Produktgasgemisch in der Regel in nennenswerten Anteilen mit der Acrylsäure in die kondensierte Phase übergeht. Erhöhte Propionsäuremengen werden im Rahmen einer heterogen katalysierten partiellen Gasphasenoxidation von z.B. Propylen und/oder Acrolein zu Acrylsäure normalerweise auch dann gebildet, wenn dabei die Reaktanden O₂ sowie Propylen und/oder Acrolein im Reaktionsgasgemisch durch erhöhte Mengen an n-Propan verdünnt werden (vgl. z.B. DE-A 10028582).

Ein Beisein von Propionaldehyd im Reaktionsgasgemisch der Partidoxidation der C₃-Vorläufer-verbindung bedingt in der Regel ebenfalls eine erhöhte Propionsäurenebenproduktbildung
(vgl. z.B. WO 2010/074177).

Aus den Schriften DE-A 10 2009 0274 01, DE-A 10 2008 041 573, DE-A 10 2008 040 799, der EP-A 1 298 120 und der EP-A 1 396 484 ist bekannt, dass dann, wenn das Reaktionsgasgemisch der heterogen katalysierten partiellen Gasphasenoxidation der C₃-Vorläuferverbindung zu Acrylsäure C₂-Verunreinigungen wie z.B. Ethylen enthält, im Rahmen der heterogen katalysierten partiellen Gasphasenoxidation im Produktgasgemisch in der Regel erhöhte Mengen des Aldehyds (monomeres) Glyoxal als Nebenprodukt auftreten und dass (monomeres) Glyoxal bei der vorstehend beschriebenen Grundabtrennung der Acrylsäure aus dem Produktgasgemisch üblicherweise in nennenswerten Anteilen mit der Acrylsäure in die kondensierte Phase übergeht.

Enthalten die Reaktionsgasgemische der heterogen katalysierten partiellen Gasphasenoxidation der C₃-Vorläuferberbindung (z.B. des Propylens) sowohl von den vorgenannten C₃-als auch C₂-Verunreinigungen, fällt bei der beschriebenen Grundabtrennung der Acrylsäure aus dem Produktgasgemisch der heterogen katalysierten partiellen Gasphasenoxidation normalerweise kondensierte Phase an, die neben Acrylsäure zusätzlich Propionsäure und Glyoxal enthält.

Aus der EP-A 770592 ist bekannt, dass geringste Mengen von in Acrylsäure enthaltenen aldehydischen Verunreinigungen wie z.B. Glyoxal die Eigenschaften der Acrylsäure signifikant beeinträchtigen können. So sollten gemäß der Lehre der EP-A 770 592 die einzelnen Aldehydanteile innerhalb einer Acrylsäure unterhalb von 1 ppm liegen, um im Rahmen der Verwendung einer solchen Acrylsäure in insbesondere radikalischen Polymerisationsreaktionen zur z.B. Herstellung von Superabsorbierenden Polymeren oder von als Dispergiermittel für Ölbohrschlamm oder als Flockungsmittel wirksamen Polymeren die optimalen Produktqualitäten zu erzielen.

Es ist allgemein bekannt (z.B. DE-A 10 028 582), dass sowohl Propionsäure als auch deren Ester mit niederen Alkanolen äußerst intensive und unangenehm riechende Geruchsträger sind, weshalb vorab einer z.B. Veresterung von Acrylsäure mit niederen Alkanolen die Propionsäure von der Acrylsäure abgetrennt werden sollte.

Die anzuwendenden Trennschritte, um aus einer im Rahmen der beschriebenen Grundabtrennung erhaltenen, das Zielprodukt Acrylsäure und die unerwünschten Nebenprodukte Glyoxal und Propionsäure enthaltenden flüssigen Phase die Acrylsäure im gewünschten Reinheitsgrad abzutrennen, können je nach Zielvorgabe sowie Art und Menge von sonstigen zusätzlich enthaltenen unerwünschten Nebenkomponenten unterschiedlichste Kombinationen von z.B. adsorptiven, extraktiven, desorptiven, destillativen, strippenden, rektifikativen, azeotrop destillativen, azeotrop rektifikativen sowie kristallisativen Verfahren sein.

Im Rahmen der vorgenannten Trennverfahren können flüssige, das Zielprodukt Acrylsäure und die unerwünschten Nebenprodukte Glyoxal und Propionsäure enthaltende Phasen unterschiedlichster Art und mit unterschiedlichen Mengenanteilen auftreten, die z.B. zwischengelagert und oder durch Wärmezufuhr thermisch belastet werden müssen.

Dies ist insofern nachteilig, als sowohl lange Verweilzeiten wie auch thermische Belastung die Wahrscheinlichkeit für eine unerwünschte radikalische Polymerisation der in der flüssigen Phase enthaltenen Acrylsäure erhöhen.

Letzteres um so mehr, als die physikalische Ähnlichkeit von Acrylsäure und Propionsäure erhöhte Verweilzeiten bei der Anwendung nicht kristallisativer thermischer Trennverfahren in der Trennvorrichtung erforderlich machen, um eine nennenswerte Trennwirkung zu erzielen, und monomeres Glyoxal die Neigung von Acrylsäure zu unerwünschter radikalischer Polymerisation erheblich ausgeprägter als andere mögliche Verunreinigungen fördert (vgl. DE-A 102008041573, DE-A 102008040799 und die DE-A 102009027401).

Es ist allgemein bekannt, dass durch Zusatz von Inhibitoren (auch Retarder genannt) zu in flüssiger Phase befindlicher Acrylsäure dem polymerisationsfördernden Einfluss von Verweilzeit und thermischer Belastung entgegengewirkt werden kann (vgl. z.B. "Polymerisationsinhibierung von (Meth-)Acrylaten, Dissertation von Dipl.-Ing. Holger Becker, Technische Universität Darmstadt, 2003").

Die Vielfalt der diesbezüglich im Stand der Technik empfohlenen Inhibitoren ist nahezu unbegrenzt (vgl. z.B. EP-A 765 856 und DE 69 701 590 T2, die einen kleinen Ausschnitt dieser Inhibitoren würdigen) und umfasst auch Verbindungen des Elements Kupfer (vgl. z.B. JP-A 2001348359).

Gemäß EP-A 1396484 (insbesondere Zeilen 16 und 17 der Spalte 2) vermag jedoch keines der bekannten Inhibitorsysteme zu befriedigen. Darüber hinaus umfasst die Mannigfaltigkeit der im Stand der Technik empfohlenen Inhibitoren gemäß der EP-A 1 396 484 (z.B. Spalte 7, Absatz [0024] sowie Spalte 1, Zeilen 40 bis 44) keine nennenswerte Bevorzugung.

Insbesondere stellt die EP-A 1 396 484 in Spalte 3, Zeilen 5 bis 10 fest, dass die bekannten Inhibitoren zwar die unerwünschte radikalische Polymerisation von Acrylsäure aufgrund thermischer Belastung derselben vergleichsweise wirksam zu hemmen vermögen, dass jedoch vor allem deren Hemmwirkung gegenüber einer Herbeiführung und/oder Förderung einer unerwünschten radikalischen Polymerisation von Acrylsäure durch in selbiger enthaltene Verunreinigungen wie Glyoxal unzureichend sei.

Eine Möglichkeit, die beschriebenen Schwierigkeiten zu überwinden, besteht darin, die Bildung von unerwünschten Nebenprodukten wie Propionsäure und Glyoxal bei der heterogen katalysierten partiellen Gasphasenoxidation von C₃-Vorläuferverbindungen (das sind Vorläuferverbindungen, die drei Kohlenstoffatome aufweisen) der Acrylsäure zu Acrylsäure zu vermeiden (z.B. durch geschickte Katalysatorwahl (vgl. z.B. JP-A 11-35519) oder durch Verwendung hochreiner C₃-Vorläuferrohstoffe (dadurch Erzeugung von z.B. weder C₂-Verunreinigungen oder n-Propan noch Cyclopropan enthaltenden Reaktionsgasgemischen; die DE-A 3521458 beschreibt z.B. die Möglichkeit der Reinigung von aus n-Propan hergestelltem Propylen und die Schriften WO 2004/018089 und WO 01/92190 beschreiben z.B. die Herstellung von Propylen aus Methanol (einer veränderten Rohstoffbasis)). Dies ist jedoch insofern nachteilig, als die diesbezüglich erforderlichen Aufwendungen die Wirtschaftlichkeit der Acrylsäureherstellung beeinträchtigen.

Vor diesem Hintergrund bestand die Aufgabe der vorliegenden Erfindung darin, ein Verfahren zur Hemmung der unerwünschten radikalischen Polymerisation von in einer flüssigen Phase P befindlicher Acrylsäure, deren Acrylsäuregehalt wenigstens 10 Gew.-% beträgt und die, bezogen auf das Gewicht der in ihr enthaltenen Acrylsäure, zusätzlich wenigsten 100 Gew.-ppm Propionsäure und wenigstens 100 Gew.-ppm Glyoxal enthält, zur Verfügung zu stellen, das insbesondere dadurch gekennzeichnet ist, dass es der Herbeiführung und/oder Förderung einer unerwünschten radikalischen Polymerisation der Acrylsäure durch das in selbiger enthaltene Glyoxal effizient entgegenwirkt.

Als Lösung der vorstehenden Aufgabe wird ein Verfahren zur Hemmung der unerwünschten radikalischen Polymerisation von in einer flüssigen Phase P befindlicher Acrylsäure, deren Acrylsäuregehalt wenigstens 10 Gew.-% beträgt und die, bezogen auf das Gewicht der in ihr enthaltenen Acrylsäure, zusätzlich wenigsten 100 Gew.-ppm Propionsäure und wenigsten 100 Gew.-ppm Glyoxal enthält, zu Verfügung gestellt, dass dadurch gekennzeichnet ist, dass der flüssigen Phase P wenigstens eine chemische Verbindung des Elements Kupfer in einer solchen Menge zugesetzt wird, dass die flüssige Phase P, bezogen auf die darin enthaltene molare Menge an Acrylsäure, 0,01 mol.-ppm bis 5mol-% an Cu zugesetzt enthält.

Das erfindungsgemäße Verfahren beruht auf dem, verglichen mit dem bisherigen Wissen des Standes der Technik, aufregenden experimentellen Befund, dass Glyoxal im Beisein von Verbindungen des Elementes Kupfer die unerwünschte radikale Polymerisation von Acrylsäure nicht mehr promoviert, sondern inhibiert (retardiert).

Durch z.B. Reaktion mit Hydroxylgruppen aufweisenden Nebenbestandteilen (z.B. H₂O, Alkohole wie Ethanol etc.) vermag monomeres Glyoxal,

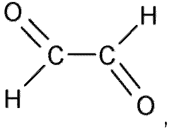

Halbacetale und/oder Acetale zu bilden. Solche Halbacetale und/oder Acetale weisen die für das monomere Glyoxal typische Polymerisationsförderwirkung in der Regel nicht mehr oder allenfalls noch in einem wesentlich geringeren Umfang wie selbiges auf.

Allerdings ist bei Halbacetalen bzw. Acetalen des Glyoxals die Bildungsreaktion häufig eine ausgesprochen reversible Reaktion, weshalb sich aus diesen Halbacetalen bzw. Acetalen, z.B. bei Einwirkung erhöhter Temperatur oder bei Entzug von Glyoxal aus dem entsprechenden Gleichgewicht, wieder monomeres Glyoxal zurückbildet, welches dann die unerwünschte radikalische Polymerisation entsprechend beeinflusst.

Im Fall von Wasser als Hydroxylgruppen aufweisendem Nebenbestandteil sind z.B. die nachfolgenden, ausgesprochen reversiblen, Acetalbildungsreaktionen bekannt (man spricht in diesem Fall auch von Hydraten des Glyoxals):

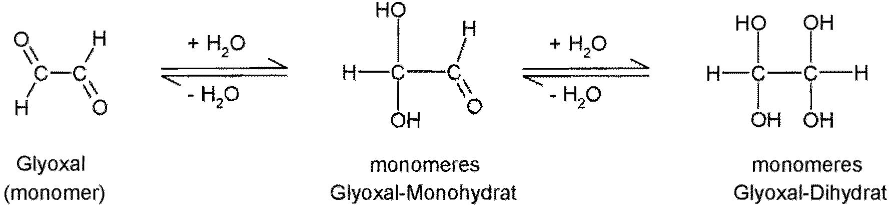

Beide vorgenannten Glyoxalhydrate bilden sich bereits unter vergleichsweise milden Bedingungen (niedere Temperaturen, beschränkte Wassergehalte sind ausreichend).

Die Begriffsbildung "monomeres" Glyoxal -Monohydrat und "monomeres" Glyoxal-Dihydrat wird dabei zum Zweck der begrifflichen Abgrenzung gegenüber "Polyglyoxal"- und "Oli-goglyoxal"-Hydraten verwendet.

In beispielhafter Weise seien nachfolgend Diglyoxal - Hydrate und Triglyoxal - Hydrate aufgeführt:

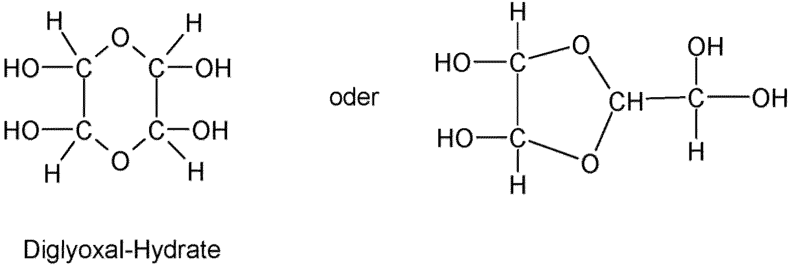

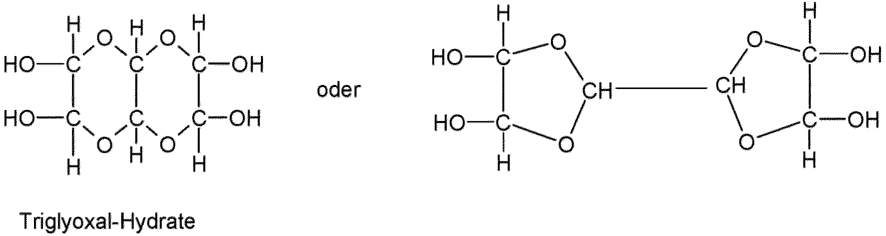

Vermutlich verläuft die Bildung der Polyglyoxal - Hydrate über das monomere Glyoxal - Dihydrat als Zwischenstufe (vgl. auch DE-A 102008041573, DE-A 102008040799 und DE-A 102009027401).

Im Unterschied zur Ausbildung der monomeren Glyoxalhydrate bedarf die Ausbildung der Polyglyoxalhydrate erhöhter Temperaturen (in der Regel erfolgt ihre Ausbildung erst bei Temperaturen oberhalb von 50° C in nennenswertem Umfang) und/oder längere Reaktionszeiten.

Daher soll aus vorgenannten Gründen in dieser Schrift der Begriff "Glyoxal" (sofern nichts anderes explizit gesagt wird, bzw. solange dem Begriff "Glyoxal" nicht explizit wenigstens eine zusätzliche Charakterisierung wie z.B. "monomeres" Glyoxal oder "Di"glyoxal - "Hydrat", oder "monomeres" Glyoxal - "Monohydrat" hinzugefügt ist) nicht nur monomeres Glyoxal, sondern auch reversibel in Form vom z.B. Acetalen und/oder Halbacetalen des monomeren Glyoxals chemisch gebundenes Glyoxal subsumieren.

Der alleinige Begriff "Glyoxal" meint also in dieser Schrift stets die Gesamtmenge aus monomerem Glyoxal und reversibel gebundenem Glyoxal.

In dieser Schrift in "Gew.-%" angegebene Gehalte an Glyoxal meinen dementsprechend stets die enthaltene Gesamtmenge aus monomerem Glyoxal und reversibel gebundenem Glyoxal, wie z.B. in monomerem Glyoxal - Monohydrat und in monomerem Glyoxal - Dihydrat, jedoch stets gerechnet als "monomeres Glyoxal" (d.h., sie meinen den Gewichtsanteil der insgesamt enthaltenen Menge an H₂C₂O₂ - Einheiten).

Dies ist für die erfindungsgemäße Verfahrensweise insbesondere insofern relevant, als Wasser normalerweise das Haupt - Nebenprodukt einer heterogen katalysierten partiellen Gasphasenoxidation einer C₃-Vorläuferverbindung von Acrylsäure zu Acrylsäure ist. Darüber hinaus wird Wasserdampf z.B. aufgrund seiner vergleichsweise erhöhten molaren Wärmekapazität häufig als Verdünnungsgas im Reaktionsgasgemisch für heterogen katalysierte partielle Gasphasenoxidationen von C₃-Vorläuferverbindungen zu Acrylsäure mit verwendet (vgl. z.B. EP-A 253 409). Bei der Grundabtrennung von Acrylsäure aus dem Produktgasgemisch einer heterogen katalysierten partiellen Gasphasenoxidation einer C₃-Vorläuferverbindung zu Acrylsäure werden daher häufig flüssige Phasen durchlaufen, die neben Acrylsäure, Propionsäure und Glyoxal auch Wasser enthalten. Grundsätzlich kann die Ausbildung von Glyoxal - Hydraten aber auch schon im Produktgasgemisch der heterogen katalysierten partiellen Gasphasenoxidation der C₃-Vorlauferverbindung der Acrylsäure erfolgen.

Im Übrigen werden Wasser bzw. wässrige Lösungen im Stand der Technik häufig auch als Absorptionsmittel für eine absorptive Grundabtrennung aus dem Produktgasgemisch der Gasphasenpartialoxidation der C₃-Vorläuferverbindung empfohlen (vgl. z.B. EP-A 1 298 120 und
US 7,332,624 B2).

Der Gehalt einer erfindungsgemäß zu behandelnden flüssigen Phase P (oder einer sonstigen flüssigen Phase) an Glyoxal (d.h., der Gesamtgehalt der flüssigen Phase P an monomerem Glyoxal und in Verbindungen wie monomerem Glyoxal - Monohydrat und monomerem Glyoxal - Dihydrat reversibel gebundenem (z.B. vermag monomeres Glyoxal auch mit Alkoholen wie Ethanol reversibel Halbacetale und/oder Acetale zu bilden) Glyoxal) wird im Sinne der vorliegenden Anmeldung wie folgt bestimmt:
Zunächst wird eine Derivatisierungslösung D hergestellt. Dazu werden 2,0 g einer 50 gew.-%igen Lösung von 2,4-Dinitrophenylhydrazin (Hersteller: Aldrich, Reinheit: ≥ 97%) bei einer Temperatur von 25 ° C in 62 ml einer 37,0 gew.-%igen wässrigen Salzsäure gelöst (Hersteller: Aldrich, Reinheit: ≥ 99,999%). Die dabei resultierende Lösung wird anschließend (ebenfalls bei einer Temperatur von 25 ° C) in 335 g destilliertes Wasser eingerührt. Nach 1-stündi-gem Rühren bei 25 ° C wird durch Abfiltrieren die Derivatisierungslösung D als das anfallende Filtrat erhalten.

Zur Bestimmung des Gehaltes einer flüssigen Phase P an Glyoxal wird 1 g (bei Bedarf kann diese Menge in entsprechender Weise erhöht werden) der Derivatisierungslösung D in ein Gewindeschraubglas eingewogen, dessen Fassungsvermögen 10 ml beträgt. Anschließend wird in das so befüllte Gewindeschraubglas eine Probe der flüssigen Phase P zugewogen, deren Menge im Bereich 0,15 bis 2,0 g liegt.

Durch Schütteln wird der Gesamtinhalt des Gewindeschraubglases anschließend gemischt und nachfolgend während eines Zeitraums von 10 Minuten bei einer Temperatur von 25 ° C sich selbst überlassen. Während dieser Zeit bildet sich aus dem im Gewindeschraubglas enthaltenen monomeren Glyoxal durch chemische Reaktion mit 2,4-Dinitrophenylhydrazin das entsprechende Hydrazon H von monomerem Glyoxal. Während dieser Zeit entzieht das 2,4-Dinitro-phenylhydrazin aber auch aus dem im Gewindeschraubglas enthaltenen monomeren Glyoxal-Monohydrat und Glyoxal-Dihydrat das in selbigen gebundene monomere Glyoxal in Form des Hydrazons H (ein entsprechender Entzug von monomerem Glyoxal aus im Gewindeschraubglas enthaltenen Polyglyoxal-Hydraten findet dagegen im Wesentlichen nicht statt).

Durch Zugabe von 0,5 g Eisessig (Hersteller: Aldrich, Reinheit: ≥ 99,8%) ins Gewindeschraubglas wird anschließend die erfolgte Hydrazonbildung eingefroren. Geht mit der Essigsäurezugabe eine Ausbildung von festem Niederschlag einher, wird sukzessive weitere Essigsäure zugegeben, um die Niederschlagbildung wieder aufzulösen (die insgesamt zugegebene Essigsäuremenge darf jedoch 1,0 g nicht überschreiten). Ist der gebildete Niederschlag auch bei Erreichen der Höchstgrenze (1,0 g) der erlaubten Essigsäuregesamtmengenzugabe nicht in Lösung gegangen, werden 0,5 g Dimethylphthalat zugewogen. Vermögen auch diese den gebildeten Niederschlag nicht aufzulösen, wird die Dimethylphthalatzugabemenge sukzessive erhöht, um diese Auflösung zu bewirken (die insgesamt zugegebene Dimethylphthalatmenge darf jedoch 1,0 g nicht überschreiten). Ist der gebildete Niederschlag auch bei Erreichen der Höchstgrenze (1,0 g) der erlaubten Dimethylphthalatgesamtmengenzugabe nicht in Lösung gegangen, werden 2 g eines Gemisches G aus 9 g Acetonitril und 1 g Dimethylphthalat zugegeben. Vermag auch diese Zugabe den Niederschlag nicht aufzulösen, wird die Zugabemenge an Gemisch G sukzessive erhöht, um diese Auflösung zu bewirken. Normalerweise überschreitet die insgesamt zugegebene Menge an Gemisch G 5 g nicht, um die Auflösung des Niederschlags zu bewirken (alle vorgenannten Auflösungsversuche werden bei 25 ° C durchgeführt).

Die wie beschrieben im Gewindeschraubglas erzeugte Lösung des Hydrazons H wird anschließend unter Anwendung der nachfolgenden Betriebsbedingungen mittels HPLC (High Pressure Liquid Chromatograpy) auf ihren Hydrazongehalt untersucht (aus der molaren Menge desselben resultiert unmittelbar die molare Menge an in der flüssigen Phase P enthaltenem Glyoxal):

| | |
|---|---|
| zu verwendende Chromatographiesäule: | Waters Symmetry C18, 150 x 4,6 mm, 5 *µ* m (der Fa. Waters Associates, Milford, Massachu setts, USA); |

Injektionsvolumen der zu analysierenden Lösung: 50 *µ* | (Zeitpunkt t = 0);
Temperatur: 40 ° C;
Eluentstrom: 1,5 ml/min;
Analysendauer: 17 min;
Equilibrierdauer: 8 min;

| | |
|---|---|
| Eluent: | im Zeitraum t > 0 min bis 15 min ein Gemisch aus 30 Gew.-% Acetonitril, 50 Gew.-% Wasser und 20 Gew.-% Tetrahydrofuran (jeweils HPLC-grade); |

| | |
|---|---|
| | im Zeitraum > 15 min bis 17 min ein Gemisch aus 65 Gew.-% Acetonitril, 30 Gew.-% Wasser und 5 Gew.-% Tetrahydrofuran; |
| | im Zeitraum > 17 min bis 25 min ein Gemisch aus 30 Gew.-% Acetonitril, 50 Gew.-% Wasser und 20 Gew.-% Tetrahydrofuran (anschließend ist die Kolonne equilibriert und wieder startbereit für die nächste Analyse). |

Die Retentionszeit des Glyoxals als Hydrazon H beträgt unter vorgenannten Bedingungen 7,613 min.

Die Analyse erfolgt mittels monochromatischer Strahlung der Wellenlänge 365 nm.

Als Analysenmethode wird die Absorptionsspektroskopie angewendet.

Die Variation des Eluents über die Eluationsdauer gewährleistet eine erhöhte Trennwirkung (in der Regel enthält die flüssige Phase P neben Glyoxal noch andere Nebenproduktaldehyde und/oder Nebenproduktketone, die mit 2,4-Dinitrophenylhydrazin das jeweilige entsprechende Hydrazon bilden).

Zur Kalibrierung des HPLC-Verfahrens wird man anwendungstechnisch zweckmäßig eine Lösung von monomerem Glyoxal in Methanol einsetzen, die 50 Gew.ppm monomeres Glyoxal enthält (vgl. DE-A 10 2008 041 573 und DE-A 10 2008 040 799).

Sie wird zu diesem Zweck wie vorstehend beschrieben mittels der Derivatisierungslösung D behandelt und anschließend der beschriebenen HPLC-Analyse unterworfen.

Als chemische Verbindungen des Elements Kupfer können für das erfindungsgemäße Verfahren sowohl solche Verbindungen eingesetzt werden, die das Kupfer in der Oxidationsstufe +2 aufweisen, als auch solche Verbindungen, in denen das Kupfer in der Oxidationsstufe +1 vorliegt, wobei ein Zusatz der erstgenannten Kupferverbindungen erfindungsgemäß bevorzugt wird.

Prinzipiell kann die der flüssigen Phase P zuzusetzende Verbindung des Elements Kupfer in der flüssigen Phase P in feinteiliger Form dispers verteilt werden (z.B. als feinteiliger Feststoff oder als dispers verteilte feinteilige Flüssigkeitströpfchen (gegebenenfalls einer die Cu-Verbindung gelöst enthaltenden Lösung)).

Erfindungsgemäß bevorzugt wird die der flüssigen Phase P zuzusetzende Kupferverbindung jedoch in der flüssigen Phase P gelöst. Die flüssige Phase P kann selbst eine Lösung oder eine flüssige Phase in einem aus mehreren flüssigen Phasen bestehenden System sein. Vorzugsweise werden beim erfindungsgemäßen Verfahren der flüssigen Phase P Salze des Kupfers zugesetzt, in denen das jeweilige Kupferkation auch komplexiert vorliegen kann.

Beispiele von für das erfindungsgemäße Verfahren geeigneten Kupferverbindungen sind Kupfer(II)phenolat, Kupfer(II)acetylacetonat, Kupfer(II)gluconat, Kupfer(II)tartrat, Kupfer(II)acetat, Kupfer(II)formiat, Kupfer(II)nitrat, Kupfer(II)hydroxid, Kupfer(II)sulfat, Kupfer(II)carbonat, Kupfer(II)naphthenat, Kupfer(II)acrylat, Kupfer(II)halogenide wie z.B. Kupfer(II)chlorid, Kupfer(II)salicylat, Kupfer(II)sulfonat, Kupfer(II)propionat, Kupfer(II)octanoat, die jeweils auch Hydratwasser aufweisen können. Ferner eignen sich Kupfer(I)-verbindungen wie CuCI, CuCN, CuJ, CuBr, Cu(I)acetat, Cu₂SO₄, Cu₂O und CuCN, aber auch Salze von komplexen Kupfer(I)-anionen wie Cu(CN)₄³⁻ oder komplexen Kupfer(I)-kationen wie Cu(NH₃)₄⁺. Als Zusatz zu wässrigen flüssigen Phasen P sind Kupfer(I)-salze weniger geeignet, da das Cu⁺ in selbigen zur Disproportionierung neigt.

Ferner eignen sich für die erfindungsgemäßen Zwecke die Kupfer(II)salze der Carbamidsäure und ihrer N-substituierten Derivate (die Herstellung entsprechender Carbamatlösungen beschreibt z.B. die DE 6961279 T2) 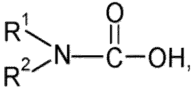 die Kupfer(II)salze der im freien Zustand nicht bekannten Thiocarbamidsäuren 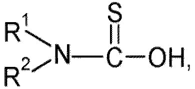 sowie insbesondere die Kupfer(II)salze der Dithiocarbamidsäuren 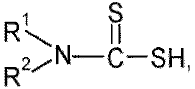 unter denen letztere erfindungsgemäß bevorzugt sind (insbesondere für wässrigen Lösungen).

In allen vorgenannten Fällen bedeuten R¹, R² unabhängig voneinander Wasserstoff oder einen organischen Rest. Bei letzterem handelt es sich erfindungsgemäß vorteilhaft um eine Methyl-, Ethyl-, Propyl-(n-Propyl- oder iso-Propyl-), Butyl-(n-Butyl-, oder iso-Butyl-, oder tert.-Butyl-), Pentyl-(n-Pentyl- oder Cyclopentyl-), Hexyl-(n-Hexyl- oder Cyclohexyl-), Methylcyclohexyl-, Benzyl-, Ethylphenyl-, Phenylethyl-, Xylyl- oder um eine Phenylgruppe. Insbesondere eignen sich für die erfindungsgemäße Verfahrensweise alle in der JP-A 2001348359 aufgeführten Kupfer(II)dithiocarbamate.

Dies sind insbesondere das Kupfer(II)dimethyldithiocarbamat, das Kupfer(II)-diethyldithiocarba-mat, das Kupfer(II)-di-n-propyldithiocarbamat, das Kupfer(II)-di-n-butyldithiocarbamat, das Kupfer(II)-di-n-pentyldithiocarbamat, das Kupfer(II)-di-n-hexyldithiocarbamat, das Kufper(II)-di-phe-nyldithiocarbamat, das Methylethyldithiocarbamat des Cu(II), das Methyl-n-propyldithiocarbamat des Cu(II), das Methyl-n-butyldithiocarbamat des Cu(II), das Methyl-n-pentyldithiocarbamat des Cu(II), das Methyl-n-hexyldithiocarbamat des Cu(II), das Mehtylphenyldithiocarbamat des Cu(II) das Ethyl-n-propyldithio-carbamat des Cu(II), das Ethyl-n-butyl-dithiocarbamat des Cu(II), das Ethyl-n-pentyldithio-carbamat des Cu(II), das Ethyl-n-hexyldithiocarbamat des Cu(II), das Ethylpehnyldithiocarbamat des Cu(II), das n-Propyl-n-butyldithiocarbamat des Cu(II), das n-Propyl-n-pentylditihiocarbamat des Cu(II), das n-Propyl-n-hexyldithiocarbamat des Cu(II), das n-Pro-pylphenyldithiocarbamat des Cu(II), das n-Butyl-n-pentyldithiocarbamat des Cu(II), das n-Butyl-n-hexyl-dithiocarbamat des Cu(II), das n-Butylphenyldithiocarbamat des Cu(II), das n-Pentyln-hexyldithiocarbamat des Cu(II), das n-Pentylphenyldithiocarbamat des Cu(II) und das n-He-ylphenyldithiocarbamat des Cu(II) sowie das Bis(2-hydroxyethyl)dithiocarbamat des Cu(II).

Selbstverständlich sind für die erfindungsgemäße Verfahrensweise auch die entsprechenden Cu(I)carbamate, -thiocarbamate und -dithiocarbamate geeignet. Besonders bevorzugt werden für die erfindungsgemäße Verfahrensweise das Dimethyldithiocarbamat, das Diethyldithiocarbamat und das Di-n-butyl-dithiocarbamat des Cu(II) verwendet. Auch eignen sich für das erfindungsgemäße Verfahren gemischte Salze des Kupfers wie Kupfer(II)dihydrocarbylthiophosphat oder Kupfer(II)dihydrocarbyldithiophosphat.

Bezogen auf die in der flüssigen Phase P enthaltene molare Menge an Acrylsäure wird man beim erfindungsgemäßen Verfahren die wenigstens eine chemische Verbindung des Elements Kupfer in solchen Mengen zusetzen, dass der Gehalt G der flüssigen Phase P an Cu, bezogen auf die darin enthaltene molare Menge an Acrylsäure 0,01 mol.-ppm bis 5 mol.-% beträgt. D.h., G kann beim erfindungsgemäßen Verfahren 0,01 mol-ppm bis 3 mol.-% oder 0,05 mol.-ppm bis 2 mol-%, oder 0,1 mol.-ppm bis 1 mol-%, oder 1 mol.-ppm bis 5000 mol.-ppm, oder 3 mol.-ppm bis 3000 mol.-ppm, oder 5 mol.-ppm bis 1000 mol.-ppm, oder 10 mol.-ppm bis 800 mol.-ppm, oder 20 mol.-ppm bis 500 mol.-ppm, oder 30 mol.-ppm bis 300 mol.-ppm, oder 40 mol.-ppm bis 200 mol.-ppm, oder 50 mol.-ppm bis 100 mol.-ppm, oder 0,1 mol.-ppm bis 10 mol.-ppm betragen.

Die wenigstens eine das Element Kupfer enthaltende chemische Verbindung kann der flüssigen Phase P z.B. als Reinsubstanz oder vorzugsweise in Lösung befindlich zugesetzt werden. Als Lösungsmittel kann z.B. flüssige Phase P selbst, oder Acrylsäure (in der Regel eine erhöhte Reinheit aufweisende Acrylsäure), oder dasjenige Lösungsmittel, in welchem die Acrylsäure in der flüssigen Phase P gelöst ist, oder ein Bestandteil oder ein Gemisch aus mehreren Bestandteilen dieses Lösungsmittels verwendet werden.

Vorzugsweise werden beim erfindungsgemäßen Verfahren Lösungen der wenigstens einen das Element Cu enthaltenden chemischen Verbindung in Acrylsäure, oder in Wasser, oder in Absorptionsmittel, in welches die Acrylsäure aus dem Produktgasgemisch der heterogen katalysierten partiellen Gasphasenoxidation der C₃-Vorläuferverbindung heraus aufgenommen wurde, der flüssigen Phase P zugesetzt.

Häufig wird die flüssige Phase P beim erfindungsgemäßen Verfahren wenigstens 20 Gew.-%, oder wenigstens 30 Gew.-%, oder wenigstens 40 Gew.-%, oder wenigstens 50 Gew.-%, oder wenigstens 60 Gew.-%, oder wenigstens 70 Gew.-%, oder wenigstens 80 Gew.-%, oder wenigstens 90 Gew.-%, oder wenigstens 95 Gew.-%, oder wenigstens 98 Gew.-% an Acrylsäure enthalten (jeweils bezogen auf das Gewicht der flüssigen Phase P).

Häufig wird die flüssige Phase P beim erfindungsgemäßen Verfahren auch Wasser enthalten. Grundsätzlich kann der Wassergehalt der flüssigen Phase P beim erfindungsgemäßen Verfahren wenigstens 1 Gew.-%, oder wenigstens 5 Gew.-%, oder wenigstens 10 Gew.-%, oder wenigstens 20 Gew.-%, oder wenigstens 30 Gew.-%, oder wenigstens 40 Gew.-%, oder wenigstens 60 Gew.-%, oder wenigstens 80 Gew.-% betragen.

Das erfindungsgemäße Verfahren ist aber insbesondere auch dann relevant, wenn die erfindungsgemäß zu behandelnde flüssige Phase P weniger als 30 Gew.-%, oder ≤ 29 Gew.-%, oder ≤ 27 Gew.-%, oder ≤ 25 Gew.-%, oder ≤ 20 Gew.-%, oder ≤ 15 Gew.-%, oder ≤ 10 Gew.-%, oder ≤ 5 Gew.-% an Wasser enthält (geringere Wassergehalte mindern die Glyoxalhydratausbildung). Vielfach wird der Wassergehalt der flüssigen Phase P jedoch ≥ 0,1 Gew.-%, oder ≥ 0,5 Gew.-%, oder ≥ 1 Gew.-% betragen (in die vorgenannten Mengenangaben ist der Wassergehalt von z.B. Glyoxalhydraten mit eingerechnet).

Häufig wird die flüssige Phase P hochsiedendes Absorptionsmittel enthalten, in das die Acrylsäure z.B. aus dem Produktgasgemisch der heterogen katalysierten partiellen Gasphasenoxidation der C₃-Vorläuferverbindung heraus aufgenommen worden ist (vgl. z.B. DE-A 102009027401).

Unter hochsiedenden Absorptionsmitteln werden in dieser Schrift Absorptionsmittel verstanden, deren Siedpunkt bei Normaldruck oberhalb desjenigen von Acrylsäure liegt. Normalerweise liegt der Siedepunkt des Absorptionsmittels bei Normaldruck (1 atm = ca. 10⁵Pa) wenigstens 20° C, vorzugsweise wenigstens 50° C, besonders bevorzugt wenigstens 75° C und ganz besonders bevorzugt wenigstens 100° C oder wenigstens125° C oberhalb des Siedepunktes von Acrylsäure (141° C bei 1 atm; im Unterschied zum Siedepunkt der Propionsäure von 141,35° C beim selben Druck; vgl. WO 2007/074045) bei dem selben Druck. Häufig liegt der Siedepunkt vorgenannter Absorptionsmittel bei Normaldruck bei Werten ≤ 400° C, häufig ≤ 350° C und vielfach auch bei ≤ 300° C oder ≤ 280° C. In besonders geeigneter Weise liegt der Siedepunkt des Absorptionsmittels bei Werten im Bereich von 200 bis 350° C (bezogen auf Normaldruck). Beispielsweise kommen als solche Absorptionsmittel alle diejenigen in Betracht, die in den Schriften
DE-A 10336386, DE-A 02449780, DE-A 19627850, DE-A 19810962, DE-A 04308087,
EP-A 0722926 und DE-A 04436243 sowie DE-A 102009027401 empfohlen werden.

In der Regel handelt es sich bei den hochsiedenden Absorptionsmitteln um organische Flüssigkeiten. Häufig bestehen sie zu wenigstens 70 Gew.-% aus solchen organischen Molekülen, die keine nach außen wirkende polare Gruppe enthalten und somit beispielsweise nicht in der Lage sind, Wasserstoffbrücken zu bilden. Besonders vorteilhafte Absorptionsmittel sind z.B. Diphenylether, Diphenyl (Biphenyl), als Diphyl ^{®} bezeichnete Gemische aus Diphenylether (70 bis 75 Gew.-%) und Diphenyl (25 bis 30 Gew.-%), sowie Dimethylphthalat, Diethylphthalat und Mischungen aus Diphyl und Dimethylphthalat bzw. Diphyl und Diethylphthalat bzw. Diphyl, Dimethylphthalat und Diethylphthalat. Eine für Absorptionszwecke ganz besonders gut geeignete Gruppe von Gemischen sind solche aus 75 bis 99,9 Gew.-% Diphyl und 0,1 bis 25 Gew.-% Dimethylphthalat und/oder Diethylphthalat.

Hochsiedende Absorptionsmittel im Sinne dieser Schrift können aber auch ionische Flüssigkeiten sein.

Beispielsweise kann die flüssige Phase P beim erfindungsgemäßen Verfahren wenigstens 1 Gew.-%, oder wenigstens 5 Gew.-%, oder wenigstens 10 Gew.-%, oder wenigstens 20 Gew.-%, oder wenigstens 30 Gew.-%, oder wenigstens 40 Gew.-%, oder wenigstens 60 Gew.-%, oder wenigstens 80 Gew.-% hochsiedendes Absorptionsmittel enthalten. Während die Verfahren des Standes der Technik danach streben, Acrylsäure im Beisein von möglichst verschwindenden Mengen an Glyoxal in die flüssige Phase zu überführen, liegt der Charme der vorliegenden Verfahrensweise nicht zuletzt darin begründet, dass sich erhöhte Glyoxalgehalte in flüssigen Phasen P bei erfindungsgemäßer Verfahrensweise hinsichtlich der Neigung der in ihnen ebenfalls enthaltenen Acrylsäure zu unerwünschter radikalischer Polymerisation als nicht mehr nachteilig sondern als vorteilhaft erweisen.

D.h., die erfindungsgemäße Verfahrensweise entfaltet ihre vorteilhafte Auswirkung insbesondere dann, wenn die flüssige Phase P, bezogen auf dass Gewicht der in ihr enthaltenen Acrylsäure, wenigstens 150 Gew.-ppm, oder wenigstens 175 Gew.-ppm, oder wenigstens 200 gew.-ppm, oder wenigstens 225 Gew.-ppm, oder wenigstens 250 Gew.-ppm, oder wenigstens 275 Gew.-ppm, oder wenigstens 300 Gew.-ppm Glyoxal enthält. Das erfindungsgemäße Verfahren eignet sich somit auch dann, wenn der in gleicher Weise bezogene Glyoxalgehalt der flüssigen Phase P > 325 Gew.-ppm, oder ≥ 350 Gew.-ppm, oder ≥ 375 Gew.-ppm, oder ≥
400 Gew.-ppm, oder ≥ 450 Gew.-ppm, oder ≥ 500 Gew.-ppm, oder ≥ 550 Gew.-ppm, oder ≥ 600 Gew.-ppm, oder ≥ 700 Gew.-ppm, oder ≥ 800 Gew.-ppm, oder ≥ 1000 Gew.-ppm, oder ≥ 1250 Gew.-ppm, oder ≥ 1500 Gew.-ppm, oder ≥ 2000 Gew.-ppm, oder ≥ 2500 Gew.-ppm beträgt.

Im Normalfall werden die wie vorstehend beschrieben bezogenen Glyoxalgehalte der flüssigen Phase P ≤ 5 Gew.-%, häufig ≤ 4 Gew.-% oder ≤ 3 Gew.-%, oft auch ≤ 2 Gew.-% oder ≤ 1 Gew.-% betragen.

In allen vorgenannten Fällen kann gleichzeitig der in entsprechender Weise bezogene (auf die enthaltende Gewichtsmenge an Acrylsäure) Gehalt der flüssigen Phase P an Propionsäure ≥ 150 Gew.-ppm, oder ≥ 175 Gew.-ppm, oder ≥ 200 Gew.-ppm, oder ≥ 225 Gew.-ppm, oder > 250 Gew.-ppm, oder > 275 Gew.-ppm, oder > 300 Gew.-ppm, oder > 325 Gew.-ppm, oder ≥ 350 Gew.-ppm, oder ≥ 375 Gew.-ppm, oder ≥ 400 Gew.-ppm, oder ≥ 450 Gew.-ppm, oder ≥ 500 Gew.-ppm, oder ≥ 550 Gew.-ppm, oder ≥ 600 Gew.-ppm, oder ≥ 700 Gew.-ppm, oder ≥ 800 Gew.-ppm, oder ≥ 1000 Gew.-ppm, oder ≥ 1250 Gew.-ppm, oder ≥ 1500 Gew.-ppm, oder > 2000 Gew.-ppm, oder ≥ 2500 Gew.-ppm betragen.

Im Normalfall werden in allen vorgenannten Fällen die wie vorstehend beschrieben bezogenen Propionsäuregehalte der flüssigen Phase P ≤ 5 Gew.-%, häufig ≤ 4 Gew.-% oder ≤ 3 Gew.-%, oft ≤ 2 Gew.-%, oder ≤ 1 Gew.-% betragen.

Selbstverständlich kann die flüssige Phase P neben Glyoxal und Propionsäure als weitere Nebenkomponenten und typische Nebenreaktionsprodukte der heterogen katalysierten partiellen Gasphasenoxidation einer C₃-Vorläuferverbindung zu Acrylsäure Verbindungen wie Fomaldehyd, Acrolein, Crotonaldehyd, Furfurale (z.B. Furfural-3, Furfural-2), Benzaldehyd, Propionaldehyd, Protoanemonin, Allylacrylat, Ameisensäure, Essigsäure, Maleinsäure, Benzoesäure und/oder Maleinsäureanhydrid enthalten (z.B. in Mengenanteilen, wie sie in der WO 2006/002713, der WO 2008/090190, der DE-A 10 2007 004960 und in der DE-A 10 2009 027401 aufgeführt sind, insbesondere in den verschiedenen flüssigen Stoffgemischen ihrer Ausführungsbeispiele).

Wie bereits erwähnt, müssen erfindungsgemäß zu behandelnde flüssige Phasen P häufig während längerer Zeitdauer gelagert werden. Während dieser Zeitdauer reagiert die Acrylsäure in gewissem Umfang mit sich selbst, und bildet durch Michael-Addition begrenzte Mengen an Diacrylsäure (vgl. z.B. WO 98/01414 und WO 2005/035478).

Das erfindungsgemäße Verfahren eignet sich daher insbesondere für flüssige Phasen P, die, bezogen auf das Gewicht der in der flüssigen Phase P enthaltenen Acrylsäure, neben den bereits aufgeführten Mengen an Glyoxal, Propionsäure und Acrylsäure zusätzlich noch ≥ 100 Gew.-ppm, oder ≥ 150 Gew.-ppm, oder ≥ 200 Gew.-ppm, oder ≥ 250 Gew.-ppm, oder ≥ 300 Gew.-ppm, oder ≥ 350 Gew.-ppm, oder ≥ 400 Gew.-ppm, oder ≥ 450 Gew.-ppm, oder ≥ 500 Gew.-ppm, oder ≥ 600 Gew.-ppm, oder ≥ 800 Gew.-ppm, oder ≥ 1000 Gew.-ppm, oder ≥ 1250 Gew.-ppm, oder ≥ 1500 Gew.-ppm, oder ≥ 1750 Gew.-ppm, oder ≥ 2000 Gew.-ppm, oder ≥ 2500 Gew.-ppm, oder ≥ 3000 Gew.-ppm, oder ≥ 4000 Gew.-ppm, oder ≥ 5000 Gew.-ppm, oder ≥ 7500 Gew.-ppm, oder ≥ 10000 Gew.-ppm an Diacylsäure enthalten.

In der Regel wird der Gehalt von erfindungsgemäß zu behandelnden flüssigen Phasen P, bezogen auf das Gewicht der darin enthaltenen Acrylsäure, an Diacrylsäure nicht mehr als 20 Gew.-%, häufig nicht mehr als 15 Gew.-% oder nicht mehr als 10 Gew.-%, sowie vielfach nicht mehr als 5 Gew.-% betragen.

Erhöhte Diacrylsäuregehalte der erfindungsgemäß zu behandelnden flüssigen Phasen P sind insofern nicht nachteilig, als Diacrylsäure bezüglich Acrylsäure selbst inhibierend wirkt (vgl. WO 2005/035478, R.C. Lamb et. al, J. Am Chem. Soc. (85), 1963, pp. 3483 - 3486 und "Polymerisationsinhibierung von (Meth)Acrylaten, Dissertation von Dipl.-Ing. Holger Becker, Technische Universität Darmstadt, 2003") und durch Anwendung und/oder Einwirkung erhöhter Temperaturen nach zuvor erfolgter Abtrennung wieder in Acrylsäure rückgespalten werden kann.

Diacrylsäuregehalte von flüssigen Phasen P sind in einfacher Weise mittels hochauflösender ¹H-NMR zu ermitteln (vgl. "Polymerisationsinhibierung von (Meth-)Acrylaten, Dissertation von Dipl.-Ing. Holger Becker, Technische Universität Darmstadt, 2003"). Das Verfahren wertet die spezifische Signalform und Signallage sowie Signalfläche der relevanten ¹H-Resonanzlinien aus. Die Bestimmung der Gehalte an Propionsäure von flüssigen Phasen P erfolgt in der Regel gaschromatographisch. Ihre Acrylsäuregehalte können ebenfalls mit ¹H-NMR, gaschromatographisch oder mit HPLC bestimmt werden.

Das erfindungsgemäße Verfahren eignet sich sowohl zur Hemmung einer unerwünschten radikalischen Polymerisation von in einer flüssigen Phase P befindlicher Acrylsäure während deren Lagerung als auch während deren prozessualen Handhabung.

Der letztere Fall liegt insbesondere dann vor, wenn die flüssige Phase P einem nicht kristallisativen thermischen Trennverfahren unterworfen wird (die dabei auftretenden Temperaturen liegen in der Regel oberhalb von 50°C, meist oberhalb von 60°C oder 70°C, oder oberhalb von 90°C oder 110°C). Dabei handelt es sich in der Regel um solche thermischen Trennverfahren, bei denen in trennwirksame Einbauten enthaltenden Trennkolonnen gasförmige (aufsteigend) und flüssige (absteigend) Stoffströme bzw. zwei flüssige Stoffströme im Gegenstrom geführt werden, wobei infolge der zwischen den Stoffströmen bestehenden Gradienten ein Wärme- und Stoffaustausch stattfindet, der letztlich die in der Trennkolonne erwünschte Trennwirkung bedingt. Beispiele für solche nicht kristallisative thermische Trennverfahren sind die Rektifikation, die azeotrope Rektifikation, die Extraktion, die Desorption, die Strippung, die Destillation, die Azeotropdestillation und die Adsorption. Da erfindungsgemäß zu behandelnde flüssige Phasen P nicht zuletzt dann entstehen, wenn das Produktgasgemisch der heterogen katalysierten partiellen Gasenphasenoxidation einer C₃-Vorläuferverbindung zu Acrylsäure einer Absorption, oder einer fraktionierenden Kondensation, oder einer partiellen Kondensation zur Grundabtrennung von Acrylsäure aus dem Produktgasgemisch unterworfen wird, eignet sich das erfindungsgemäße Verfahren auch zur Polymerisationsinhibierung von im Rahmen solcher thermischer Trennverfahren auftretender flüssiger Phasen P. Selbstredend eignet sich das erfindungsgemäße Verfahren der Polymerisationsinhibierung auch dann, wenn die flüssige Phase P einem kristallisativen thermischen Trennverfahren unterworfen wird.

Der Begriff "thermische Trennverfahren" soll dabei zum Ausdruck bringen, dass dem System zur Erzielung der gewünschten Trennwirkung Wärme zugeführt oder entzogen werden muss (vgl. DE-A 10 2008 041573 und DE-A 10 2008 8040799).

Die prozessual zu behandelnde flüssige Phase P kann dabei die erfindungsgemäß zuzusetzende, wenigstens eine chemische Verbindung des Elements Kupfer bereits von Beginn des thermischen Trennverfahrens an zugesetzt enthalten (d.h., es kann dem thermischen Verfahren bereits erfindungsgemäß behandelt zugeführt werden). Selbstverständlich kann die wenigstens eine chemische Verbindung des Elements Kupfer aber auch erst im Verlauf des thermischen Trennverfahrens zugesetzte werden (z.B. bei einer Rektifikation in der Rücklaufflüssigkeit gelöst, oder bei einer Absorption im Absorptionsmittel gelöst, oder bei einer fraktionierenden Kondensation in der Rücklaufflüssigkeit gelöst, oder bei einer Direktkühlung des Produktgasgemisches der heterogen katalysierten partiellen Gasphasenoxidation der C₃-Vorläuferverbindung in der zur Direktkühlung eingesetzten Quenchflüssigkeit gelöst).

Selbstverständlich muss die der flüssigen Phase P erfindungsgemäß zuzusetzende wenigstens eine chemische Verbindung des Elements Kupfer nicht das einzige der flüssigen Phase P zugesetzte Inhibitorsystem sein. Vielmehr kann die flüssige Phase P zusätzlich einen oder mehrere Inhibitoren aus der Gruppe umfassend die Nitroxyl-Radikale (auch als N-Oxyl-Radikal bezeichnet) (z.B. die in der DE-A 19734171 offenbarten wie 4-Hydroxy-2,2,6,6-tetramethylpiperidin-1-oxyl oder 1,4-Dihydroxy-2,2,6,6,-tetramethylpiperidin), Phenothiazine wie z.B. Dibenzo-1,4-thiazin (Phenothiazin), phenolische Verbindungen wie Hydrochinon, 2,4-Dimethyl-6-t-butylphenol und Hydrochinonmonomethylether, molekularer Sauerstoff, Cersalze wie z.B. Cer(III)salze, Mangansalze (z.B. Mangan(III)salze wie Mangan(III)acetat-Dihydrat und Mangan(III)-Di-n-butyldi-thiocarbamat, p-Phenylendiamine (z.B. die in der DE-A 19734171 offenbarten), organische Nitrosoverbindungen wie 4-Nitrosophenol (und die anderen in der DE-A 19734171 offenbarten), Methylenblau und alle anderen z.B. in der EP-A 765856 offenbarten Inhibitoren zugesetzt enthalten. Vorgenannte Inhibitoren können der flüssigen Phase P in entsprechenden Mengen zugesetzt sein, wie sie in dieser Schrift für die wenigstens eine der flüssigen Phase P zuzusetzende Kupfer enthaltende Verbindung ausgeführt sind und empfohlen werden.

Bei der Durchführung von nicht kristallisativen thermischen Trennverfahren an erfindungsgemäß behandelten flüssigen Phasen P in trennwirksame Einbauten (z.B. Trennböden wie Dual-Flow-Böden) eingebaut enthaltenden Trennkolonnen kann als zusätzliche Inhibiermaßnahme als Quelle für molekularen Sauerstoff z.B. Luft oder mit Stickstoff angereicherte Luft (Magerluft) durch die Trennkolonne (z.B. eine Rektifikationskolonne oder Absorptionskolonne) geströmt werden, wie es z.B. in der DE-A 102009027401 und in der DE-A 102007004960 praktiziert wird.

Solche thermischen Trennverfahren (z.B. alle in der WO 2011/000808 A2, in der DE-A 10336386, in der DE-A 19924532, in der DE-A 19924533, und in der DE-A 102007004960 beschriebenen thermischen Trennverfahren) werden erfindungsgemäß bevorzugt in Vorrichtungen durchgeführt, die den Empfehlungen der US 6441228 B2 und der US 6966973 B2 entsprechen.

Eine günstige Inhibitorkombination zur Stabilisierung der in der DE-A 102009027401 ausgeführten Rektifikation des Acrylsäure enthaltenden Absorbats A* in der Rektifikationskolonne K30 umfasst (neben der die Rektifikationkolonne K30 durchströmenden Luft), bezogen auf die jeweils zu stabilisierende (zu inhibierende) Menge an Acrylsäure, z.B. 0,1 bis 3 mol.-ppm Cu (zugesetzt in Form wenigstens einer Cu enthaltenden Verbindung (vorzugsweise Cu(II)-Di-n-butyldithiocarbamat) und 50 bis 1000 Gew.-ppm Phenothiazin, vorzugsweise 0,2 bis 2 mol.-ppm an Cu und 100 bis 500 Gew.-ppm Phenothiazin und besonders bevorzugt 0,3 bis 1 mol.-ppm Cu und 200 bis 400 Gew.-ppm Phenothiazin. Die Inhibitorzufuhr in die Rektifikationskolonne K30 erfolgt mit Vorteil über die Rücklaufflüssigkeit in selbiger gelöst, sowie über das der Rektifikationskolonne K30 zugeführte Absorbat A* in selbigem gelöst.

Der Vorteil der erfindungsgemäßen Verfahrensweise besteht, wie bereits gesagt, unter anderem darin, dass auf dem Weg zur Erzeugung einer flüssigen Phase P bei der heterogen katalysierten partiellen Gasphasenoxidation einer C₃-Vorläuferverbindung der Acrylsäure nicht von hochreinen C₃-Vorläuferverbindungen der Acrylsäure ausgegangen werden muss.

Beispielsweise kann für die heterogen katalysierte partielle Gasphasenoxidation zur Herstellung von Acrylsäure ein Reaktionsgasausgangsgemisch eingesetzt werden, das, bezogen auf die in ihm enthaltene molare Menge an der eingesetzten C₃-Vorläuferverbindung (z.B. Propan, Propylen, Acrolein, Propionsäure, Propionaldehyd, Propanol und/oder Glyzerin, unter denen Propylen und Acrolein bevorzugt sind), eine molare Gesamtmenge an C₂-Verbindungen (z.B. Ethan, Ethylen, Acetylen, Acetaldehyd, Essigsäure und/oder Ethanol) von ≥ 100 mol.ppm, oder ≥ 150 mol.ppm, oder ≥ 200 mol.ppm, oder ≥ 250 mol.ppm, oder ≥ 300 mol.ppm, oder ≥ 350 mol.ppm, oder ≥ 400 mol.ppm, oder > 450 mol.ppm, oder > 500 mol.ppm, oder ≥ 600 mol.ppm, oder ≥ 750 mol.ppm, oder ≥ 1000 mol.ppm, oder ≥ 1250 mol.ppm, oder ≥ 1500 mol.ppm, oder ≥ 2000 mol.ppm, oder ≥ 2500 mol.ppm, oder ≥ 3000 mol.ppm aufweist.

In der Regel wird die vorgenannte molare Gesamtmenge an C₂-Verbindungen im Reaktionsgasausgangsgemisch der heterogen katalysierten partiellen Gasphasenoxidation der C₃-Vorläuferverbindung zu Acrylsäure (in gleicher Weise bezogen) nicht mehr als 30 mol-%, meist nicht mehr als 20 mol-% oder als 10 mol-%, häufig nicht mehr als 5 mol-% betragen.

Darüber hinaus kann für die heterogen katalysierte partielle Gasphasenoxidation zur Herstellung von Acrylsäure auf dem Weg zur Herstellung der flüssigen Phase P ein Reaktionsgasausgangsgemisch eingesetzt werden, das, bezogen auf die in ihm enthaltene Menge an der eingesetzten C₃-Vorläuferverbindung (z.B. Propan, Propylen, Acrolein, Propionsäure, Propionaldehyd, Propanol, und/oder Glyzerin, unter denen Propylen und Acrolein bevorzugt sind) zusätzlich zu den vorgenannten Gesamtmengen an C₂-Verbindungen eine molare Gesamtmenge an Cyclopropan von ≥ 10 mol.-ppb (ppm = part per million; ppb = part per billion), oder ≥ 25 mol.ppb, oder ≥ 50 mol.ppb, oder ≥ 75 mol.ppb, oder ≥ 100 mol.ppb, oder ≥ 1 mol.ppm, oder ≥ 10 mol.ppm, ≥ 20 mol.ppm, oder ≥ 30 mol.ppm, oder ≥ 50 mol.ppm, oder ≥ 75 mol.ppm, oder ≥ 100 mol.ppm, oder ≥ 150 mol.ppm, oder ≥ 250 mol.ppm, oder ≥ 300 mol.ppm, oder ≥ 400 mol.ppm, oder ≥ 500 mol.ppm, oder ≥ 750 mol.ppm, oder ≥ 1000 mol.ppm, oder ≥ 1500 mol.ppm, oder ≥ 2000 mol.ppm an Cyclopropan enthalten. In der Regel werden die wie vorstehend bezogenen Cyclopropan-Gehalte des Reaktionsgasausgangsgemischs für die heterogen katalysierte partielle Gasphasenoxidation der C₃-Vorläuferverbindung zu Acrylsäure nicht mehr als 5 mol-%, häufig nicht mehr als 3 mol-% oder als 2 mol-% und vielfach nicht mehr als 1 mol-% betragen.

Ferner kann das für die heterogen katalysierte partielle Gasphasenoxidation zur Herstellung von Acrylsäure eingesetzte Reaktionsgasausgangsgemisch z.B. im Fall von Propylen oder Acrolein als C₃-Vorläuferverbindung (aber auch im Fall der anderen von n-Propan verschiedenen C₃-Vorläuferverbindungen), bezogen auf die Gewichtsmenge des enthaltenen Propylen bzw. Acrolein (der von n-Propan verschiedenen C₃-Vorläuferverbindung) ≥ 0,05 Gew.-% n-Propan, oder ≥ 0,1 Gew.-% n-Propan, oder ≥ 0,2 Gew.-% n-Propan, oder ≥ 0,3 Gew.-% n-Propan, oder ≥ 0,4 Gew.-% n-Propan, oder ≥ 0,5 Gew.-% n-Propan, oder ≥ 0,75 Gew.-% n-Propan, oder ≥ 1 Gew.-% n-Propan, oder ≥ 2 Gew.-% n-Propan, oder ≥ 3 Gew.-% n-Propan, oder ≥ 5 Gew.-% n-Propan, oder ≥ 6 Gew.-% n-Propan, oder ≥ 10 Gew.-% n-Propan, oder ≥ 20 Gew.-% n-Propan enthalten. Üblicherweise enthält das Reaktionsgasausgangsgemisch einer heterogen katalysierten partiellen Gasphasenoxidation von Propylen und/oder Acrolein (der von n-Propan verschiedenen C₃-Vorläuferverbindung) zu Acrylsäure jedoch nicht mehr als 80 Vol.-%, häufig nicht mehr al 70 Vol.-% und vielfach nicht mehr als 60 Vol.-% (meist aber nicht weniger als
0,1 Vol.-%) an n-Propan.

Der Begriff "Reaktionsgasausgangsgemisch", meint in allen vorgenannten Fällen dasjenige Gasgemisch, das dem Katalysatorbett zum Zweck der Partialoxidation der in ihm enthaltenen C₃-Vorläuferverbindung zu Acrylsäure zugeführt wird. Neben der C₃-Vorläuferverbindung, unerwünschten Verunreinigungen sowie molekularem Sauerstoff als Oxidationsmittel enthält das Reaktionsgasausgangsgemisch in der Regel noch inerte Verdünnungsgase wie z. B. N₂, CO₂, H₂O, Edelgas, molekularen Wasserstoff etc.. Jedes inerte Verdünnungsgas ist normalerweise so beschaffen, dass es zu wenigstens 95 mol-% seiner Ausgangsmenge im Verlauf der heterogen katalysierten Partialoxidation unverändert erhalten bleibt.

Der Anteil der C₃-Vorläuferverbindung am Reaktionsgasausgangsgemisch kann z. B. im Bereich von 4 bis 20 Vol.-%, oder von 5 bis 15 Vol.-%, oder von 6 bis 12 Vol.-% liegen.

Normalerweise enthält das Reaktionsgasausgangsgemisch, bezogen auf die Stöchiometrie der Partialoxidationsreaktion der C₃-Vorläuferverbindung zu Acrylsäure, einen Überschuss an molekularem Sauerstoff, um die in der Regel oxidischen Katalysatoren wieder zu reoxidieren.

Dieser Überschuss kann im Fall einer nachfolgenden Anwendung der erfindungsgemäßen Verfahrensweise besonders hoch gewählt werden, da mit zunehmendem Sauerstoffüberschuss in der Regel auch eine Zunahme der unerwünschten Nebenkomponentenbildung an Glyoxal einhergeht.

In gleicher Weise kann bei der heterogen katalysierten partiellen Gasphasenoxidation der C₃-Vorläuferverbindung zur Acrylsäure die im Katalysatorbett vorliegende maximale Reaktionstemperatur vergleichsweise erhöht gewählt werden, wenn im Anschluss an die Partialoxidation das erfindungsgemäße Verfahren zur Anwendung kommt. Dies ist u. a. darauf zurückzuführen, dass mit zunehmender Maximaltemperatur in der Regel auch eine Zunahme der unerwünschten Nebenkomponentenbildung an Glyoxal einhergeht. Die Anwendung erhöhter Maximaltemperaturen gestattet in der Regel jedoch den Einsatz von Katalysatoren mit geringerer Aktivität, was die Möglichkeit einer verlängerten Katalysatorstandzeit eröffnet. Allerdings erfolgt bei Verwendung von Katalysatoren mit geringerer Aktivität mit zunehmendem Umsatz der C₃-Vorläuferverbindung in zunehmendem Umfang häufig auch unerwünschte Vollverbrennung derselben. Als Zwischenprodukt kann dabei gegebenenfalls ebenfalls Glyoxal gebildet werden.

In ähnlicher Weise kann im Kontext mit der erfindungsgemäßen Verfahrensweise auch bei der Auswahl der Belastung des Katalysatorbetts mit C₃-Vorläuferverbindung großzügiger verfahren werden.

Des Weiteren hat sich gezeigt, dass die Glyoxalnebenproduktbildung durch erhöhte Wasserdampfgehalte im Reaktionsgasgemisch begünstigt wird. Das erfindungsgemäße Verfahren ist deshalb nicht zuletzt dann von Relevanz, wenn das für die heterogen katalysierte partielle Gasphasenoxidation der C₃-Vorläuferverbindung eingesetzte Reaktionsgasausgangsgemisch ≥ 1 Gew.-%, oder ≥ 2 Gew.-%, oder ≥ 3 Gew.-%, oder ≥ 4 Gew.-%, oder ≥ 5 Gew.-%, oder ≥ 7 Gew.-%, oder ≥ 9 Gew.-%, oder ≥ 15 Gew.-%, oder ≥ 20 Gew.-% an Wasserdampf enthält. Im Regelfall wird der Wasserdampfgehalt des Reaktionsgasausgangsgemischs jedoch nicht mehr als 40 Gew.-%, häufig nicht mehr als 30 Gew.-% betragen.

Im Übrigen kann das Verfahren der heterogen katalysierten partiellen Gasphasenoxidation zur Herstellung der Acrylsäure in an sich bekannter Weise wie im Stand der Technik beschrieben durchgeführt werden.

Handelt es sich bei der C₃-Vorläuferverbindung z. B. um Propylen und/oder Acrolein, kann die heterogen katalysierte partielle Gasphasenoxidation z. B. wie in den Schriften WO 2005/042459, WO 2005/047224 und WO 2005/047226 beschrieben durchgeführt werden.

Ist die C₃-Vorläuferverbindung z. B. Propan, kann die heterogen katalysierte partielle Gasphasenoxidation zur Herstellung der Acrylsäure z. B. wie in den Schriften EP-A 608 838, DE-A 198 35 247, DE-A 102 45 585 und DE-A 102 46 119 beschrieben durchgeführt werden.

Ist die C₃-Vorläuferverbindung z. B. Glycerin, kann die heterogen katalysierte partielle Gasphasenoxidation zur Herstellung der Acrylsäure z. B. wie in den Schriften WO 2007/090991, WO 2006/114506, WO 2005/073160, WO 2006/114506, WO 2006/092272 oder WO 2005/073160 beschrieben durchgeführt werden.

Es wurde auch schon vorgeschlagen, das Propylen als C₃-Vorläuferverbindung durch eine der partiellen Gasphasenoxidation vorgeschaltete partielle Dehydrierung und/oder Oxidehydrierung von Propan zu erzeugen (z. B. WO 076370, WO 01/96271, EP-A 117146, WO 03/011804 und WO 01/96270).

Das erfindungsgemäße Verfahren ist insbesondere auch dann vorteilhaft anwendbar, wenn das in der flüssigen Phase P enthaltene Glyoxal zu wenigstens 20 mol-%, oder zu wenigstens 30 mol-%, oder zu wenigstens 50 mol-%, oder zu wenigstens 70 mol-%, oder zu wenigstens 90 mol-%, oder zu wenigstens 95 mol-% als monomeres Glyoxal-Monohydrat und/oder monomeres Glyoxal-Dihydrat in der flüssigen Phase P vorliegt (bzw. in der flüssigen Phase P enthalten ist).

Das erfindungsgemäße Verfahren ist nicht zuletzt dann günstig, wenn die erfindungsgemäß zu behandelnde flüssige Phase P auf ein Produktgasgemisch einer heterogen katalysierten partiellen Gasphasenoxidation eines C₃-Vorläufers der Acrylsäure zurückgeht, das, bezogen auf die im Produktgasgemisch enthaltene molare Menge an Acrylsäure, wenigstens 100, oder wenigstens 150, oder wenigstens 200 mol.ppm Glyoxal, oder ≥ 250 mol.ppm Glyoxal, oder ≥ 300 mol.ppm Glyoxal, oder ≥ 400 mol.ppm Glyoxal, oder ≥ 500 mol.ppm Glyoxal, oder ≥ 750 mol.ppm Glyoxal, oder ≥ 1000 mol.ppm Glyoxal, oder ≥ 1250 mol.ppm Glyoxal, oder ≥ 1500 mol.ppm Glyoxal enthält (zur Bestimmung der vorgenannten, auf die enthaltene molare Menge an Acrylsäure bezogenen, Glyoxalgehalte des Produktgasgemischs wird man durch Abkühlen desselben wenigstens die darin enthaltene Acrylsäure, die darin enthaltenen Halbacetale und/oder Acetale des Glyoxals sowie das darin enthaltene monomere Glyoxal in die kondensierte Phase überführen und selbige anschließend möglichst zeitnah zu ihrer Erzeugung wie in dieser Schrift für eine flüssige Phase P beschrieben auf ihren Gehalt an Glyoxal und Acrylsäure analysieren).

Dies vor allem dann, wenn das Produktgasgemisch gleichzeitig den vorgenannten Mengenanteilen entsprechende Propionsäuregehalte aufweist.

Im Normalfall werden die vorgenannten Propionsäure- und Glyoxalgehalte des Produktgasgemischs (in gleicher Weise bezogen) ≤ 5 mol-% betragen. Vielfach wird der Acrylsäuregehalt der vorgenannten Produktgasgemische 1 bis 30 Vol.-% betragen.

Häufig werden erfindungsgemäß zu behandelnde flüssige Phasen P auch einer azeotropen Rektifikation zur Abtrennung von darin enthaltenem Wasser unterworfen. Als diesbezüglich geeignete Schleppmittel kommen insbesondere Heptan, Dimethylcyclohexan, Ethylcyclohexan, Toluol, Ethylbenzol, Octan, Chlorbenzol, Xylol oder Mischungen derselben (z.B. 60 Gew.-% Toluol und 40 Gew.-% Heptan) in Betracht. Als alternative Schleppmittel können aber auch Methylisobutylketon oder Isopropylacetat eingesetzt werden. Im Übrigen kann wie in den Schriften EP-A 778255, EP-A 695736 und US 2004/0242826 beschrieben verfahren werden. Erfindungsgemäß zu behandelnde flüssige Phasen P sind daher insbesondere auch solche flüssigen Phasen P, die wenigstens eines der vorgenannten Schleppmittel und Wasser enthalten. In der Regel beträgt der Wassergehalt solcher flüssiger Phasen P wenigstens 10 Gew.-% und der Gehalt an azeotropem Schleppmittel wenigstens 1 Gew.-%, häufig wenigstens 2 Gew.-% oder wenigstens 5 Gew.-%.

Das erfindungsgemäße Verfahren ist nicht zuletzt auch dann relevant, wenn aus einer erfindungsgemäß behandelten flüssigen Phase P darin enthaltenes Glyoxal und enthaltene Propionsäure kristallisativ abgetrennt werden, wobei sich das Glyoxal und die Propionsäure in der verbleibenden Mutterlauge und die Acrylsäure im Kristallisat anreichern, und von der Mutterlauge in wenigsten einen der Verfahrensschritte rückgeführt wird, mit Hilfe derer die erfindungsgemäß behandelte flüssige Phase P aus dem Produktgasgemisch der heterogen katalysierten partiellen Gasphasenoxidation der C₃-Vorläuferverbindung erzeugt (hergestellt) wurde. Das kristallisative Abtrennverfahren kann dabei in entsprechender Weise durchgeführt werden, wie es in den Schriften DE-A 102008041573, DE-A 102008040799 und WO 2007/074044 sowie DE-A 102007029053 beschrieben ist.

Korrosionsuntersuchungen haben ergeben, dass für mit wenigstens einer das Element Cu enthaltenden chemischen Verbindung erfindungsgemäß inhibierte flüssige Phasen P der DIN Werkstoff 1.4571 ein geeigneter und in voll befriedigender Weise korrosionsbeständiger Apparatewerkstoff ist.

US Provisional Application No. 61/391102 wurde am 8. Oktober 2010 eingereicht. Im Hinblick auf die obengenannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

Beispiele und Vergleichbeispiele
1. Herstellung von mit unterschiedlichen Polymerisationsinhibitoren versetzten flüssigen Phasen P sowie von unterschiedlich inhibierten flüssigen Vergleichsphasen.

Wie in der DE-A 102007055086 beschrieben frisch hergestellte Reinacrylsäure, die, bezogen auf ihr Gewicht, mit 200 Gew.-ppm Methoxyphenol (MEHQ) polymerisationsinhibiert war, wurde unter vermindertem Druck (1000 Pa) durch zweifaches, aufeinanderfolgend durchgeführtes Überdestillieren von MEHQ befreit.

Die Reinheit des so erzeugten Reinacrylsäuredestillats RD betrug > 99,8 Gew.-%, bei einem Aldehyd- und Ketongesamtgehalt < 5 Gew.-ppm, einem Diacrylsäuregehalt < 1 Gew.-ppm und einem Propionsäuregehalt von 190 Gew.-ppm.

Aus dem Reinacrylsäuredestillat RD wurde eine Teilmenge in identische Proben von 1 ml aufgeteilt.

Aus einer anderen Teilmenge wurden verschiedene Stammlösungen erzeugt, in welchen z.B. unterschiedliche Mengen unterschiedlicher Polymerisationsinhibitoren gelöst wurden. Aus einer weiteren Teilmenge des Reinacrylsäuredestillats wurde eine mit Propionsäure (Reinheit > 99,5 Gew.-%) versetzte Stammlösung erzeugt.

In entsprechender Weise wurde eine Glyoxal enthaltende Stammlösung erzeugt. Die dazu verwendete Glyoxalquelle war eine wässrige Lösung der Fa. Aldrich, die gemäß Herstellerinformation 40 Gew.-% Glyoxal gelöst enthielt (gerechnet als monomeres Glyoxal). Mit der Herstellung dieser Stammlösung ging eine geringfügige Niederschlagsbildung einher, die vermutlich auf höhermolekulare, in der Reinacrylsäure RD schwerlösliche Polyglyoxale (bzw. Hydrate derselben) zurückzuführen war. Es wurde daher von der Niederschlagsbildung abfiltriert und nachfolgend wie in dieser Schrift beschrieben der Glyoxalgehalt des Filtrats bestimmt (60 g Reinacrylsäuredestillat RD wurden bei 25° C mit 1 g der 40 gew.-%igen wässrigen Glyoxallösung versetzt; anschließend wurde während 15 min bei 25° C gerührt; dann wurde der gebildete Niederschlag abfiltriert).

Aus den Stammlösungen entnommene Probemengen wurden mit dem Reinacrylsäuredestillat RD in der jeweils erforderlichen Menge verdünnt und mit den Verdünnungen die verschiedenen 1 ml Proben wie gewünscht dotiert. Anschließend wurden die dotierten Proben durch Einfrieren konserviert. Einige Proben wurden bei 35° C sich selbst überlassen, um ihren Gehalt an Diacrylsäure zu erhöhen.

2. Untersuchung der Polymerisationsneigung der proben der verschiedenen flüssigen Phasen.

Zur Untersuchung der Polymerisationsneigung der jeweiligen dotierten Probe wurde selbige wieder verflüssigt und jeweils ein HPLC-Vial (transparentes Gefäß mit 1,5 ml Füllvolumen) mit 0,5 ml der jeweiligen Probe unter Luft befüllt und anschließend mit einer Bördelkappe dicht verschlossen. Unmittelbar nach Fertigstellung wurden jeweils bis zu sechs wie beschrieben befüllte Vials in eine dafür angefertigte Halterung eingehängt und bei einer Temperatur von 120° C in einem Umlufttrockenschrank sich selbst überlassen, während die Halterung mit sechs Umdrehungen pro Minute rotierte, um eine vollständige Durchmischung in den Vials zu gewährleisten (sechsmal pro Minute kam der flüssige Inhalt des jeweiligen Vials mit der Bördelkappe in Berührung). Dann wurde die Zeit T bis zur vollständigen Polymerisation der jeweiligen Probe im zugehörigen Vial erfasst. Dazu wurden die Proben in den Vials im Trockenschrank mit Hilfe einer Videokamera (die maximale Filmlaufzeit war 720 Minuten) überwacht und der Videofilm nachträglich visuell ausgewertet.

Jeder Versuch wurde dreimal wiederholt und die zugehörigen Werte für T arithmetisch gemittelt. Die resultierenden Mittelwerte 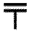 (in Minuten) für die verschiedenen Proben, einschließlich ihrer zugehörigen relevanten Gehalte an von Acrylsäure verschiedenen Bestandteilen, sind nachfolgend aufgelistet (die Mengenangaben sind jeweils bezogen auf die in der jeweiligen Probe enthaltene Menge an Acrylsäure).

Dabei wurden folgende Abkürzungen verwendet:

| | | | | | |
|---|---|---|---|---|---|
| PA | = | Propionsäure | Mn (Ac)₃ | = | Mn(III)acetat |
| DA | = | Diacrylsäure | NiSO₄ | = | Ni(II)sulfat |
| | | | Nadetc | = | Natriumdiethyldithiocarbamat |
| Gly | = | Glyoxal | CuGluc | = | Cu(II)gluconat |
| PTZ | = | Phenothiazin | CuTart | = | Cu(II)tartratmonohydrat |
| Cu(Ac)₂ | = | Cu(II)acetat | CuAcetyl | = | Cu(II)Acetylacetonat |
| CuAc | = | Cu(I)acetat | Cudetc | = | Cu(II)-diethyldithiocarbamat |
| Ce(Ac)₃ | = | Cer(III)acetat | Cudbtc | = | Cu(II)-di-n-butyldithiocarbamat |
| Fe(Ac)₂ | = | Fe(II)acetat | Cub(2he)dtc | = | Cu(II)-bis(2-hydroxyethyl)dithiocarbamat |

| Probe | 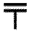 (min) |
|---|---|
| RD mit 190 Gew.-ppm PA | 20 |
| RD mit 190 Gew.-ppm PA, 50 Gew.-ppm Gly | 16 |
| RD mit 190 Gew.-ppm PA, 10 Gew.-ppm PTZ | 162 |
| RD mit 190 Gew.-ppm PA, 5 Gew.-ppm Gly, 10 Gew.-ppm PTZ | 130 |
| RD mit 190 Gew.-ppm PA, 80 Gew.-ppm Gly, 10 Gew.-ppm PTZ | 99 |
| RD mit 190 Gew.-ppm PA, 1,52 mol.-ppm Cu (als Cudbtc) | 70 |
| RD mit 190 Gew.-ppm PA, 5 Gew.-ppm Gly, 1,52 mol.-ppm Cu (als Cudbtc) | 80 |
| RD mit 190 Gew.-ppm PA, 80 Gew.-ppm Gly, 1,52 mol.-ppm Cu (als Cudbtc) | 105 |
| RD mit 190 Gew.-ppm PA, 200 Gew.-ppm Gly, 1,52 mol.-ppm Cu (als Cudbtc) | 142 |
| RD mit 190 Gew.-ppm PA, 500 Gew.-ppm Gly, 1,52 mol.-ppm Cu (als Cudbtc) | > 720 |
| RD mit 300 Gew.-ppm PA, 64 Gew.-ppm DA, 500 Gew.-ppm Gly, 1,52 mol.-ppm Cu(als Cudbtc) | > 720 |
| RD mit 190 Gew.-ppm PA, 3,97 mol.-ppm Cu (als Cu(Ac)₂) | 113 |
| RD mit 190 Gew.-ppm PA, 250 Gew.-ppm Gly, 10 Gew.-ppm PTZ, 3,97 mol.-ppm Cu (als Cu(Ac)₂) | 270 |
| RD mit 190 Gew.-ppm PA, 500 Gew.-ppm Gly, 10 Gew.-ppm PTZ, 3,97 mol.-ppm Cu (als Cu(Ac)₂) | 353 |
| RD mit 190 Gew.-ppm PA, 10 Gew.-ppm PTZ, 3,97 mol.-ppm Cu (als Cu(Ac)₂) | 161 |
| RD mit 190 Gew.-ppm PA, 100 Gew.-ppm Gly, 10 Gew.-ppm PTZ | 84 |
| RD mit 190 Gew.-ppm PA, 100 Gew.-ppm Gly, 10 Gew.-ppm PTZ, 0,76 mol.-ppm Cu (als Cudbtc) | 125 |
| RD mit 190 Gew.-ppm PA, 100 Gew.-ppm Gly, 10 Gew.-ppm PTZ, 1,52 mol.-ppm Cu (als Cudbtc) | 139 |
| RD mit 190 Gew.-ppm PA, 100 Gew.-ppm Gly, 10 Gew.-ppm PTZ, 3,80 mol.-ppm Cu (als Cudbtc) | 157 |
| RD mit 190 Gew.-ppm PA, 100 Gew.-ppm Gly, 10 Gew.-ppm PTZ, 7,60 mol.-ppm Cu (als Cudbtc) | 180 |
| RD mit 190 Gew.-ppm PA, 100 Gew.-ppm Gly, 10 Gew.-ppm PTZ, 15,2 mol.-ppm Cu (als Cudbtc) | 196 |
| RD mit 190 Gew.-ppm PA, 100 Gew.-ppm Gly, 10 Gew.-ppm PTZ, 76 mol.-ppm Cu (als Cudbtc) | 228 |

| Probe | 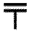 (min) |
|---|---|
| RD mit 190 Gew.-ppm PA, 100 Gew.-ppm Gly, 10 Gew.-ppm PTZ, 152 mol.-ppm Cu (als Cudbtc) | 311 |
| RD mit 190 Gew.-ppm PA, 500 Gew.-ppm Gly, 10 Gew.-ppm PTZ | 61 |
| RD mit 190 Gew.-ppm PA, 500 Gew.-ppm Gly, 10 Gew.-ppm PTZ, 3,42 mol.-ppm Cu (als Cudbtc) | 440 |
| RD mit 190 Gew.-ppm PA, 500 Gew.-ppm Gly, 10 Gew.-ppm PTZ, 18,9 Gew.-ppm Nadetc | 50 |
| RD mit 190 Gew.-ppm PA, 1000 Gew.-ppm Gly, 10 Gew.-ppm PTZ, 1,90 mol.-ppm Cu (als Cu(I)Ac) | 335 |
| RD mit 190 Gew.-ppm PA, 1000 Gew.-ppm Gly, 10 Gew.-ppm PTZ, 1,90 mol.-ppm Cu (als CuGluc) | 204 |
| RD mit 190 Gew.-ppm PA, 1000 Gew.-ppm Gly, 10 Gew.-ppm PTZ, 1,70 mol.-ppm Cu (als CuAcetyl) | 246 |
| RD mit 190 Gew.-ppm PA, 1000 Gew.-ppm Gly, 10 Gew.-ppm PTZ, 1,50 mol.-ppm Cu (als CuTart) | 179 |
| RD mit 190 Gew.-ppm PA, 500 Gew.-ppm Gly, 10 Gew.-ppm PTZ, 3,70 mol.-ppm Cu (als Cub(2he)dtc) | 439 |
| RD mit 190 Gew.-ppm PA, 10 Gew.-ppm PTZ, 10 Gew.-ppm NiSO₄ | 142 |
| RD mit 190 Gew.-ppm PA, 500 Gew.-ppm Gly, 10 Gew.-ppm PTZ, 10 Gew.-ppm NiSO₄ | 38 |
| RD mit 190 Gew.-ppm PA, 500 Gew.-ppm Gly, 10 Gew.-ppm NiSO₄ | 15 |
| RD mit 190 Gew.-ppm PA, 10 Gew.-ppm Gly, 10 Gew.-ppm PTZ, 10 Gew.-ppm Ce(Ac)₃ | > 720 |
| RD mit 190 Gew.-ppm PA, 500 Gew.-ppm Gly, 10 Gew.-ppm PTZ, 10 Gew.-ppm Ce(Ac)₃ | 161 |
| RD mit 190 Gew.-ppm PA, 500 Gew.-ppm Gly, 10 Gew.-ppm Ce(Ac)₃ | 33 |
| RD mit 190 Gew.-ppm PA, 10 Gew.-ppm PTZ, 10 Gew.-ppm Fe(Ac)₂ | 125 |
| RD mit 190 Gew.-ppm PA, 500 Gew.-ppm Gly, 10 Gew.-ppm PTZ, 10 Gew.-ppm Fe(Ac)₂ | 36 |
| RD mit 190 Gew.-ppm PA, 500 Gew.-ppm Gly, 10 Gew.-ppm Fe(Ac)₂ | 10 |
| RD mit 190 Gew.-ppm PA, 10 Gew.-ppm PTZ, 10 Gew.-ppm Mn(Ac)₃ | > 720 |
| RD mit 190 Gew.-ppm PA, 500 Gew.-ppm Gly, 10 Gew.-ppm PTZ, 1 Gew.-ppm Mn(Ac)₃ | 265 |
| RD mit 190 Gew.-ppm PA, 500 Gew.-ppm Gly, 10 Gew.-ppm Mn(Ac)₃ | 12 |
| R D mit 625 Gew.-ppm PA, 119 Gew.-ppm DA, 500 Gew.-ppm Gly, 10 Gew.-ppm PTZ, 3,97 mol.-ppm Cu (als | 490 |

| | |
|---|---|
| Probe | 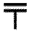 (min) |
| Cu(Ac)₂) | |

Eine entsprechende Retardwirkung wie für Glyoxal im Beisein von Kupfer enthaltenden Verbindungen konnte in entsprechenden Dotierversu chen auch für Hydroxyaceton, Glycolaldehyd, Glycerinaldehyd, 1,3-Dihydroxyaceton und Glutardialdehyd nachgewiesen werden. 3-Hydroxybenz-aldehyd verfügt bereits für sich eine Retardwirkung auf Acrylsäure. Salicylaldehyd und o-Phthaldialdehyd, die ebenso wie 3-Hydroxybenzaldehyd Nebenprodukt einer heterogen katalysierten Partialoxidation von C₃-Vorläuferverbindungen zu Acrylsäure sein können, fördern hingegen die unerwünschte radikalische Polymerisation von Acrylsäure.

## Patentansprüche

1. Verfahren zur Hemmung der unerwünschten radikalischen Polymerisation von in einer flüssigen Phase P befindlicher Acrylsäure, deren Acrylsäuregehalt wenigstens 10 Gew.-% beträgt und die, bezogen auf das Gewicht der in ihr enthaltenen Acrylsäure, zusätzlich wenigstens 100 Gew.-ppm Propionsäure und wenigstens 100 Gew.-ppm Glyoxal enthält, **dadurch gekennzeichnet, dass** der flüssigen Phase P wenigstens eine chemische Verbindung des Elements Kupfer in einer solchen Menge zugesetzt wird, dass die flüssige Phase P, bezogen auf die darin enthaltene molare Menge an Acrylsäure, 0,01 mol.-ppm bis 5 mol-% an Cu zugesetzt enthält.

2. Verfahren nach Anspruch 1 **dadurch gekennzeichnet, dass** die wenigstens eine chemische Verbindung das Kupfer in einer der Oxydationsstufen +2, +1 aufweist.

3. Verfahren nach einem der Ansprüche 1 bis 2, **dadurch gekennzeichnet, dass** die der flüssigen Phase P zugesetzte wenigstens eine chemische Verbindung des Elements Kupfer in der flüssigen Phase P dispers verteilt und / oder in der flüssigen Phase P gelöst wird.

4. Verfahren nach einem der Ansprüche 1 bis 3, **dadurch gekennzeichnet, dass** die flüssige Phase P wenigstens 1 Gew.-% Wasser enthält.

5. Verfahren nach einem der Ansprüche 1 bis 4, **dadurch gekennzeichnet, dass** die flüssige Phase P, bezogen auf das Gewicht der in ihr enthaltenen Acrylsäure, ≥ 100 Gew.-ppm Diacrylsäure enthält.

6. Verfahren nach einem der Ansprüche 1 bis 5, **dadurch gekennzeichnet, dass** die in der flüssigen Phase P enthaltene Acrylsäure das Produkt einer heterogen katalysierten Partialoxidation einer C₃ -Vorläuferverbindung der Acrylsäure ist, bei dem das für die Partialoxidation eingesetzte, die C₃ -Vorläuferverbindung enthaltende, Reaktionsgasausgangsgemisch, bezogen auf die in ihm enthaltene molare Menge der Cs-Vorläuferverbindung, eine molare Gesamtmenge an C₂-Verbindungen von ≥ 100 mol. ppm aufweist.

7. Verfahren nach einem der Ansprüche 1 bis 6, **dadurch gekennzeichnet, dass** die in der flüssigen Phase P enthaltene Acrylsäure das Produkt einer heterogen katalysierten Partialoxidation einer C₃-Vorläuferverbindung der Acrylsäure ist, bei dem das für die Partialoxidation eingesetzte, die C₃-Vorläuferverbindung enthaltende, Reaktionsgasausgangsgemisch, bezogen auf die in ihm enthaltene molare Menge der Cs-Vorläuferverbindung, eine molare Gesamtmenge an Cyclopropan von ≥ 10 mol. ppb aufweist.

8. Verfahren nach einem der Ansprüche 1 bis 7, **dadurch gekennzeichnet, dass** die in der flüssigen Phase P enthaltene Acrylsäure das Produkt einer heterogen katalysierten Partialoxidation einer C₃-Vorläuferverbindung der Acrylsäure ist, bei dem das für die Partialoxidation eingesetzte, die C₃-Vorläuferverbindung enthaltende Reaktionsgasausgangsgemisch bis zu 80 Vol.-% n-Propan enthält.

9. Verfahren nach einem der Ansprüche 6 bis 8, **dadurch gekennzeichnet, dass** die C₃-Vorläuferverbindung Propylen, Acrolein oder n-Propan ist.

10. Verfahren nach einem der Ansprüche 1 bis 9, **dadurch gekennzeichnet, dass** die wenigstens eine chemische Verbindung des Elements Kupfer ein Salz ist.

## Claims

1. A process for inhibiting unwanted free-radical polymerization of acrylic acid present in a liquid phase P which has an acrylic acid content of at least 10% by weight and which, based on the weight of the acrylic acid present therein, additionally comprises at least 100 ppm by weight of propionic acid and at least 100 ppm by weight of glyoxal, wherein at least one chemical compound of the element copper is added to the liquid phase P in such an amount that the liquid phase P, based on the molar amount of acrylic acid present therein, comprises 0.01 molar ppm to 5 mol% of added Cu.

2. The process according to claim 1, wherein the at least one chemical compound has the copper in one of the oxidation states +2, +1.

3. The process according to claim 1 or 2, wherein the at least one chemical compound of the element copper added to the liquid phase P is dispersed in the liquid phase P and/or dissolved in the liquid phase P.

4. The process according to any of claims 1 to 3, wherein the liquid phase P comprises at least 1% by weight of water.

5. The process according to any of claims 1 to 4, wherein the liquid phase P, based on the weight of the acrylic acid present therein, comprises ≥ 100 ppm by weight of diacrylic acid.

6. The process according to any of claims 1 to 5, wherein the acrylic acid present in the liquid phase P is the product of a heterogeneously catalyzed partial oxidation of a C₃ precursor compound of acrylic acid, in which the starting reaction gas mixture which is used for the partial oxidation and comprises the C₃ precursor compound, based on the molar amount of the C₃ precursor compound present therein, has a total molar amount of C₂ compounds of ≥ 100 molar ppm.

7. The process according to any of claims 1 to 6, wherein the acrylic acid present in the liquid phase P is the product of a heterogeneously catalyzed partial oxidation of a C₃ precursor compound of acrylic acid, in which the starting reaction gas mixture which is used for the partial oxidation and comprises the C₃ precursor compound, based on the molar amount of the C₃ precursor compound present therein, has a total molar amount of cyclopropane of ≥ 10 molar ppb.

8. The process according to any of claims 1 to 7, wherein the acrylic acid present in the liquid phase P is the product of a heterogeneously catalyzed partial oxidation of a C₃ precursor compound of acrylic acid, in which the starting reaction gas mixture which is used for the partial oxidation and comprises the C₃ precursor compound comprises up to 80% by volume of n-propane.

9. The process according to any of claims 6 to 8, wherein the C₃ precursor compound is propylene, acrolein or n-propane.

10. The process according to any of claims 1 to 9, wherein the at least one chemical compound of the element copper is a salt.

## Revendications

1. Procédé d'inhibition de la polymérisation par voie radicalaire non souhaitée d'acide acrylique se trouvant dans une phase liquide P, dont la teneur en acide acrylique est d'au moins 10% en poids et qui contient, par rapport au poids de l'acide acrylique qu'elle contient, en plus au moins 100 ppm en poids d'acide propionique et au moins 100 ppm en poids de glyoxal, **caractérisé en ce que** la phase liquide P est additionnée d'au moins un composé chimique de l'élément cuivre en une quantité telle que la phase liquide P contient, sous forme ajoutée, par rapport à la quantité molaire d'acide acrylique qu'elle contient, 0,01 ppm en mole à 5 ppm en mole de Cu.

2. Procédé selon la revendication 1, **caractérisé en ce que** ledit au moins un composé chimique présente le cuivre dans un des étages d'oxydation +2, +1.

3. Procédé selon l'une des revendications 1 à 2, **caractérisé en ce que** ledit au moins un composé chimique de l'élément cuivre ajouté à la phase liquide P est réparti de manière dispersée dans la phase liquide P et/ou est dissous dans la phase liquide P.

4. Procédé selon l'une des revendications 1 à 3, **caractérisé en ce que** la phase liquide P contient au moins 1% en poids d'eau.

5. Procédé selon l'une des revendications 1 à 4, **caractérisé en ce que** la phase liquide P contient, par rapport au poids de l'acide acrylique qu'elle contient, ≥ 100 ppm en poids de di(acide acrylique).

6. Procédé selon l'une des revendications 1 à 5, **caractérisé en ce que** l'acide acrylique contenu dans la phase liquide P est le produit d'une oxydation partielle catalysée par voie hétérogène d'un composé précurseur en C₃ de l'acide acrylique, pour lequel le mélange réactionnel de départ, contenant le composé précurseur en C₃, utilisé pour l'oxydation partielle présente, par rapport à la quantité molaire qu'il contient de composé précurseur en C₃, une quantité molaire totale de composés en C₂ ≥ 100 ppm en mole.

7. Procédé selon l'une des revendications 1 à 6, **caractérisé en ce que** l'acide acrylique contenu dans la phase liquide P est le produit d'une oxydation partielle catalysée par voie hétérogène d'un composé précurseur en C₃ de l'acide acrylique, pour lequel le mélange réactionnel de départ, contenant le composé précurseur en C₃, utilisé pour l'oxydation partielle présente, par rapport à la quantité molaire qu'il contient de composé précurseur en C₃, une quantité molaire totale de cyclopropane ≥ 10 ppb en mole.

8. Procédé selon l'une des revendications 1 à 7, **caractérisé en ce que** l'acide acrylique contenu dans la phase liquide P est le produit d'une oxydation partielle catalysée par voie hétérogène d'un composé précurseur en C₃ de l'acide acrylique, pour lequel le mélange réactionnel de départ, contenant le composé précurseur en C₃ utilisé pour l'oxydation partielle contient jusqu'à 80% en volume de n-propane.

9. Procédé selon l'une des revendications 6 à 8, **caractérisé en ce que** le composé précurseur en C₃ est du propylène, de l'acroléine ou du n-propane.

10. Procédé selon l'une des revendications 1 à 9, **caractérisé en ce que** ledit au moins un composé chimique de l'élément cuivre est un sel.
